# EUROPEAN PATENT SPECIFICATION

(11) **EP 2 655 613 B1**
(45) Date of publication and mention of the grant of the patent: **15.02.2017**
(21) Application number: 11851176.5
(22) Date of filing: 22.12.2011
(51) Int. Cl.: A61K 39/00, A61K 39/295, A61P 31/16, A61P 37/04, C07K 14/005, C07K 14/11, C12N 7/00, C12N 15/82

(54) **VIRUS LIKE PARTICLE PRODUCTION IN PLANTS**
PRODUKTION VIRUSÄHNLICHER PARTIKEL IN PFLANZEN
PRODUCTION DE PARTICULES DE TYPE VIRAL CHEZ DES VÉGÉTAUX

(30) Priority: 22.12.2010 US 201061426401 P; 13.06.2011 US 201161496371 P
(43) Date of publication of application: 30.10.2013
(73) Proprietor: Medicago Inc., Québec, Québec G1V 3V9 (CA)
(72) Inventor: D'AOUST, Marc-Andre, Quebec Quebec G1X 4N4 (CA); COUTURE, Manon, St. Augustin de Desmaures Quebec G3A 2A5 (CA); LAVOIE, Pierre-Olivier, Quebec Quebec G1N 2L7 (CA); VEZINA, Louis-Philippe, Neuville Quebec G0A 2R0 (CA)
(74) Representative: van Kooij, Adriaan
(86) International application number: PCT/CA2011/001427
(87) International publication number: WO 2012/083445

(56) References cited:
- WO-A1-2009/009876
- WO-A1-2009/076778
- WO-A1-2010/003225
- WO-A1-2010/077712
- WO-A2-2008/061243
- HAYNES JOEL R: "Influenza virus-like particle vaccines", EXPERT REVIEW OF VACCINES ENGLAND, EXPERT REVIEWS LTD, GB, vol. 8, no. 4, 1 April 2009 (2009-04-01), pages 435-445, XP009128169, ISSN: 1744-8395, DOI: 10.1586/ERV.09.8
- D'AOUST MARC-ANDRÉ ET AL: "Influenza virus-like particles produced by transient expression in Nicotiana benthamiana induce a protective immune response against a lethal viral challenge in mice", PLANT BIOTECHNOLOGY JOURNAL, BLACKWELL, OXFORD, GB, vol. 6, no. 9, 1 December 2008 (2008-12-01), pages 930-940, XP002598510, ISSN: 1467-7644
- D'AOUST MARC-ANDRE ET AL: "The production of hemagglutinin-based virus-like particles in plants: a rapid, efficient and safe response to pandemic influenza", PLANT BIOTECHNOLOGY JOURNAL, BLACKWELL, OXFORD, GB, vol. 8, no. 5, 1 June 2010 (2010-06-01), pages 607-619, XP002598511, ISSN: 1467-7644, DOI: 10.1111/J.1467-7652.2009.00496.X
- MESHCHERYAKOVA, Y. A. ET AL.: 'Cowpea Mosaic Virus Chimeric Particles Bearing the Ectodomain of Matrix Protein 2 (M2E) of the Influenza A Virus: Production and Characterization' MOLECULAR BIOLOGY vol. 43, 2009, pages 685 - 694, XP055112546
- NEMCHINOV, L. G. ET AL.: 'Transient expression of the ectodomain of matrix protein (M2e) of avian influenza a virus in plants' PROTEIN EXPRESSION AND PURIFICATION vol. 56, 2007, pages 153 - 159, XP022317081

## Description

### FIELD OF INVENTION

The present invention relates to producing chimeric virus proteins in plants. More specifically, the present invention also relates to producing virus-like particles comprising chimeric virus proteins in plants.

### BACKGROUND OF THE INVENTION

Vaccination provides protection against disease caused by a like agent by inducing a subject to mount a defense prior to infection. Conventionally, this has been accomplished through the use of live attenuated or whole inactivated forms of the infectious agents as immunogens. To avoid the danger of using the whole virus (such as killed or attenuated viruses) as a vaccine, recombinant viral proteins, for example subunits, have been pursued as vaccines. Both peptide and subunit vaccines are subject to a number of potential limitations. Subunit vaccines may exhibit poor immunogenicity, owing to incorrect folding, poor antigen presentation, or differences in carbohydrate and lipid composition. A major problem is the difficulty of ensuring that the conformation of the engineered proteins mimics that of the antigens in their natural environment. Suitable adjuvants and, in the case of peptides, carrier proteins, must be used to boost the immune response. In addition these vaccines elicit primarily humoral responses, and thus may fail to evoke effective immunity. Subunit vaccines are often ineffective for diseases in which whole inactivated virus can be demonstrated to provide protection.

Virus-like particles (VLPs) are potential candidates for inclusion in immunogenic compositions. VLPs closely resemble mature virions, but they do not contain viral genomic material. Therefore, VLPs are nonreplicative in nature, which make them safe for administration as a vaccine. In addition, VLPs can be engineered to express viral glycoproteins on the surface of the VLP, which is their most native physiological configuration. Moreover, since VLPs resemble intact virions and are multivalent particulate structures, VLPs may be more effective in inducing neutralizing antibodies to the glycoprotein than soluble envelope protein antigens.

VLPs for over thirty different viruses have been generated in insect and mammalian systems for vaccine purpose (Noad, R. and Roy, P., 2003, Trends Microbiol 11: 438-44). Several studies have demonstrated that recombinant influenza proteins self-assemble into VLPs in cell culture using mammalian expression plasmids or baculovirus vectors (e.g. Gomez-Puertas et al., 1999, J. Gen. Virol, 80, 1635-1645; Neumann et al., 2000, J. Virol., 74, 547-551; Latham and Galarza, 2001, J. Virol., 75, 6154-6165).

Gomez-Puertas et al. (1999, J. Gen. Virol., 80, 1635-1645) demonstrated that efficient formation of influenza VLPs depends on the expression levels of viral proteins. Neumann et al. (2000, J. Virol., 74, 547-551) established a mammalian expression plasmid-based system for generating infectious influenza virus-like particles entirely from cloned cDNAs. Latham and Galarza (2001, J. Virol., 75, 6154-6165) reported the formation of influenza VLPs in insect cells infected with recombinant baculovirus co-expressing HA, NA, M1, and M2 genes. This study demonstrated that influenza virion proteins self-assemble upon co-expression in eukaryotic cells and that the M1 matrix protein was required for VLP production.

In several expression systems, including baculovirus, vaccinia virus, drosophila (DS-2) cells, Vero cells and yeast spheroblasts, expression of Pr^{55gag} from the human immunodeficiency virus (HIV) results in assembly and release of virus-like particles (VLPs), similar in morphology to immature HIV virions (reviewed by Deml et al., 2005, Molecular Immunology 42: 259-277).

HIV envelope protein gp160 can be incorporated into Gag-derived VLPs. However, only a limited number of envelope proteins are incorporated despite high expression of Pr^{55gag}. Wang et al. showed that replacement of the transmembrane domain and cytosolic tail domains (TM/CT) of the HIV envelope protein by those of another viral envelope protein, including influenza hemagglutinin resulted in an increase in incorporation of envelope protein into Pr^{55gag} derived VLPs (Journal of Virology, 2007, 81: 10869-10878). Chimeric HIV envelope protein comprising HA TM/CT was also shown to be incorporated into influenza M1-derived VLPs when co-expressed in insect cells using a baculovirus expression system (WO2008/005777).

Influenza virus penetration into a cell depends on HA-dependent receptor-mediated endocytosis. The influenza virus infection cycle is initiated by the attachment of the virion surface HA protein to a sialic acid-containing cellular receptor (glycoproteins and glycolipids). The neuraminidase (NA) protein mediates processing of sialic acid receptors. In the acidic confines of internalized endosomes containing an influenza virion, the HA protein undergoes conformational changes that lead to fusion of viral and cell membranes, virus uncoating, and M2-mediated release of M1 proteins from nucleocapsid-associated ribonucleoproteins (RNPs), which migrate into the cell nucleus for viral RNA synthesis. Latham and Galarza, 200, J. Virol. 75,6154- 6165) reported the formation of influenza VLPs in insect cells infected with recombinant baculovirus co-expressing HA, NA, M1, and M2 genes. Furthermore, Gomez-Puertas et al., 2000, J Virol. 74, 11538-11547) teach that, in addition to the hemaglutinin (HA), the matrix protein (M1) of the influenza virus is essential for VLP budding from insect cells. However, Chen et al. (2007, J. Virol. 81, 7111-7123) teach that M1 may not be required for VLP formation.

Most characterized budding mechanisms use a vacuolar protein sorting pathway (VPS) pathway of the host (see Chen and Lamb, Virology 372, 2008). Many enveloped viruses have been shown to interact with proteins of the VPS pathway, requiring the action of endosomal sorting complex required for transport (ESCRT) protein complexes (see Table I of Chen and Lamb 2008). The late protein domain that interacts with proteins of the VPS pathway is found on core and matrix proteins of viruses, and therefore VPS-dependant budding requires the presence of matrix or core proteins. Palmitoylation of the cytosolic tail of influenza HA is required for budding but the mechanism is not well understood and involvement of other surface protein domains may be involved. The minimal requirements for budding remain unknown and the participation of the ectodomain in this process cannot be ruled out. Additionally, the VPS pathway in plants is poorly understood (see Schellmann S., and Pimpl P., Current Op Plant Biol 12:670-676, 200).

Budding of influenza is known to be independent of the VPS pathway. Budding of influenza virus involves Rab11 pathway (Bruce et al., J. Virol 84:5848-5859, 2010). Rab proteins are GTPase anchors positioned at the surface of transport vesicles inside cells, and they are involved in vesicle formation from the donor compartment, transport, docking and fusion to acceptor compartment (Vazquez-Martinez and Malagon Frontiers in Endocrinology 2:1-9, 2011). Components of the Rab11 pathway have been identified in plants. However, the trafficking components of plants have evolved leading to several specific features of the plant's endomembrane system, including for example a large and specialized vacuole, rapid movement of Golgi stacks and unique organization of endosomal compartments, and an expanded number of Rab GTPases (Rojo E., and Deneke J., Plant Phys 147:1493-1503, 2008). Influenza particle or virus budding is dependent on Rab11 (Bruce et. al., J. Virol 84:5848-5859, 2010) but the protein or protein domain that interacts with Rab11 or Rab11 associated proteins have not been identified. However, the minimal domain or domains of HA that may be required for the budding process and VLP production are unknown.

In plants, HIV Pr^{55gag} accumulates at extremely low levels if not engineered for accumulation in chloroplasts (Meyers et al., 2008, BMC Biotechnology 8:53; Scotti et al., 2010, Planta 229: 1109-1122). Accumulation in chloroplast, however, is incompatible with the incorporation of correctly folded HIV envelope protein since the maturation and folding of the latter requires post-translational modifications specific of the secretion pathway. Rybicki et al. (2010, Plant Biotechnology Journal 8: 620-637) notes that "...it seems that no one has successfully expressed whole HIV Env gp160 protein or even the majority of the protein, in plants at reasonable yield...".

The rabies virus (RV) is a member of the family Rhabdoviridae. Like most members of this family, RV is a non-segmented, negative stranded RNA virus whose genome codes for five viral proteins: RNA-dependent RNA polymerase (L); a nucleoprotein (N); a phosphorylated protein (P); a matrix protein (M) located on the inner side of the viral protein envelope; and an external surface glycoprotein (G). Dietzschold B et al. (1991), Crit. Rev. Immunol. 10: 427-439.

Cell-cultured based vaccines for rabies are limited to growing inactivated strains of the virus in cell cultures. These vaccines comprise the virus grown in cell cultures. Current biotechnological approaches aim at expressing the coat protein gene of the rabies virus to develop a safe recombinant protein that could be deployed as an active vaccine. Stable expression of rabies virus glycoprotein has been shown in Chinese Hamster Ovary cells (Burger et al., 1991). A full length, glycosylated protein of 67 K that co-migrated with the G-protein isolated from virus-infected cells, was obtained.

WO/1993/001833 teaches production of a virus like particles (VLPs) in a baculovirus expression system containing an RNA genome including a 3' domain and a filler domain surrounded by a sheath of rabies M protein and rabies M1 protein. The VLP also includes a lipid envelope of rabies G protein.

WO 2008/061243 discloses respiratory syncytial virus-virus like particles (VLPS) that express and/or contains RSV proteins.

The Varicella Zoster virus (VZV), also known as human herpesvirus 3 (HHV-3), is a member of the alphaherpesvirus subfamily of the Herpesviridae family of viruses. VLPs expressing glycoproteins or tegument proteins have previously been generated from different herpesvirus family members. Light particles (L-particles) comprised of enveloped tegument proteins, have been obtained from cells infected with either herpes simplex virus type 1 (HSV-1), equine herpesvirus type 1 (EHV-1), or pseudorabies virus (McLauchlan and Rixon (1992) J. Gen. Virol. 73: 269-276; U.S. Pat. No. 5,384,122). A different type of VLP, termed pre-viral DNA replication enveloped particles (PREPs), could be generated from cells infected with HSV-1 in the presence of viral DNA replication inhibitors. The PREPs resembled L-particles structurally, but contained a distinct protein composition (Dargan et al. (1995) J. Virol. 69: 4924-4932; U.S. Pat. No. 5,994,116). Hybrid VLPs expressing fragments of the gE protein from VZV have been produced by a technique using protein p1 encoded by the yeast Ty retrotransposon (Garcia-Valcarcel et al. (1997) Vaccine 15: 709-719; Welsh et al. (1999) J. Med. Virol. 59: 78-83; U.S. Pat. No. 6,060,064). US 2011/0008838 describes chimeric VLPs that comprise at least one VZV protein, but does not comprise a yeast Ty protein. The chimeric VLPs comprise a viral core protein such as influenza M1 or Newcastle disease protein M and at least one varicella zoster virus (VZV) protein.

The spread of a newly evolved coronavirus (CoV) caused a global threat of severe acute respiratory syndrome (SARS) pandemics in 2003 (Kuiken, T. et al., 2003, Lancet 362: 263-270). As with other coronaviruses, SARS-CoV has the morphology of enveloped particles with typical peripheral projections, termed "corona" or "spikes," surrounding the surface of a viral core (Ksiazek, T. G. et al., 2003 , N Engl J Med 348: 1953-1966; Lin, Y. et al., 2004, Antivir Ther 9: 287-289). Outside the coronavirus particle core is a layer of lipid envelope containing mainly three membrane proteins, the most abundant M (membrane) protein, the small E (envelope) protein, and the S (spike) protein. The homo-trimers of the S protein collectively form the aforementioned corona, which is involved in viral binding to host receptors, membrane fusion for viral entry, cell-to-cell spread and tissue tropism of coronaviruses.

Baculovirus expression systems have been used to produce SARS VLPs (Ho, Y. et al., 2004, Biochem Biophys Res Commun 318: 833-838; Mortola, E. and Roy, P., 2004, FEBS Lett 576: 174-178). However, due to the intrinsic differences between insect cells and mammalian cells, the VLPs assembled in the insect (SF9) cells exhibited a size of 110 nm in diameter, which is much larger than the 78 nm of the authentic SARS-CoV virions (Lin, Y. et al., 2004, supra, and Ho, Y. et al., 2004, supra). Moreover, immunogenicity of the insect cell-based SARS-VLP remains uninvestigated. Other researchers also tried to use mammalian expression systems to produce SARS VLPs (Huang, Y. et al., 2004, J Virol 78: 12557-65). However, the extracellular release of VLPs is not efficient, and the yield of VLPs is not satisfying. For example WO/2005/035556 describes a system for making SARS-CoV-virus-like particles (SARS-CoV-VLPs) comprising one or more recombinant vectors which express the SARS-CoV E-protein, the SARS-CoV M-protein, and the SARS-CoV S-protein in mammalian cells.

Formation of VLPs, in any system, places considerable demands on the structure of the proteins - altering stretches of sequence of a protein may not have much of an effect on the expression of the polypeptide itself, however structural studies are lacking to demonstrate the effect of such alterations on the formation of VLPs. The cooperation of the various regions and structures of the protein has evolved with the virus and may not be amendable to similar alterations without loss of VLP formation.

To improve VLPs as vaccine candidates other expression system beside insect and mammalian cells need to be explored. There is therefore a need to assess the ability of the plant expression system to produce chimeric protein VLPs. In particular, there is a need to identify the minimal number of virus proteins which will assemble into VLPs and to evaluate the morphology and immunogenicity of those VLPs.

### SUMMARY OF THE INVENTION

The present invention relates to producing chimeric virus proteins in plants. More specifically, the present invention also relates to producing virus-like particles comprising chimeric virus proteins in plants.

The present invention provides a method of producing a virus like particle (VLP) in a plant comprising,
a) introducing a nucleic acid comprising a regulatory region active in the plant and operatively linked to a chimeric nucleotide sequence encoding, in series, an ectodomain from a virus trimeric surface protein or fragment thereof, fused to an influenza hemagglutinin transmembrane domain and cytoplasmic tail, into the plant, or portion of the plant, the ectodomain is derived from a virus of a family of Retroviridae, Rhabdoviridae, Coronaviridae or Filoviridae and
b) incubating the plant or portion of the plant under conditions that permit the expression of the nucleic acid, thereby producing the VLP.

The method as described above may further comprising a step (c) of harvesting the plant and purifying the VLP. Furthermore, the VLP may not contain a viral matrix or a core protein.

The ectodomain from the virus trimeric surface protein may be derived for example from the genus Lentivirus, Lyssavirus, Coronavirus or Ebolavirus. The ectodomain from the virus trimeric surface protein may be derived from for example, but not limited to, HIV, Rabies virus, RSV, SARS virus or Ebola virus. The virus trimeric surface protein in it native form may comprise an ectodomain and a transmembrane domain/cytoplasmic tail, for example but not limited to F protein (RSV, Measles, Mumps, Newcastle Disease), S protein (SARS), env protein (HIV), G protein (rabies), envelope glycoprotein including E, B, C, I, H (VZV, cytomegalovirus, herpesvirus, Epstein-barr virus), GP glycoprotein (ebola, marburg), hemagglutinin (variola virus, vaccinia virus).

The present invention also includes the method as described above, wherein the influenza transmembrane domain and cytoplasmic tail is obtained from H5 (A/Indonesia/05/2005) or H3 (A/Brisbane/10/2007). The transmembrane domain and cytoplasmic tail may comprise the nucleotide sequence defined in SEQ ID NO:41, or SEQ ID NO:42.

The present invention also provides the method as descried above, wherein in the step of introducing (step a), the nucleic acid is transiently expressed in the plant. Alternatively, in the step of introducing (step a), the nucleic acid may be stably expressed in the plant.

The present invention also includes the method as described above, wherein, in the step of introducing (step a), one or more than one additional nucleic acid, selected from the group of a nucleotide sequence encoding one or more than one chaperone protein, proton channel protein, protease inhibitor, or a combination thereof, is introduced to the plant.

The present invention provides a VLP produced by the method described above. The chimeric virus trimeric surface protein of the VLP may comprise plant-specific N-glycans, or modified N-glycans. The VLP may also comprise one or more than one lipid derived from the plant.

The present invention includes a composition comprising an effective dose of the VLP as described above for inducing an immune response, and a pharmaceutically acceptable carrier.

The present invention relates to producing chimeric virus trimeric surface protein from viruses of the Retroviridae, Rhabdoviridae, Herpesviridae, Coronaviridae or Filoviridae family and producing virus-like particles comprising these chimeric virus trimeric surface protein in plants.

Furthermore, the present invention relates to producing human immunodeficiency virus (HIV), Rabies virus, Varicella Zoster Virus (VZV), Severe acute respiratory syndrome (SARS) virus or Ebola virus chimeric trimeric surface protein in plants. The present invention relates to producing HIV, Rabies, VZV, SARS and Ebola chimeric virus-like particles in plants.

According to the present invention there is provided a method of producing chimeric HIV, rabies, VZV, SARS or Ebola VLPs in a plant comprising introducing a nucleic acid encoding a chimeric HIV, rabies, VZV, SARS or Ebola virus protein operatively linked to a regulatory region active in the plant, into the plant, or portion of the plant, and incubating the plant or portion of the plant under conditions that permit the expression of the nucleic acid, thereby producing the chimeric HIV, rabies, VZV, SARS or Ebola VLPs.

The present invention further provides for a VLP comprising a chimeric HIV rabies, VZV, SARS or Ebola protein. The VLP may be produced by the method as provided by the present invention. The HIV, rabies, VZV, SARS or Ebola VLP may also be produced within a plant.

Chimeric VLPs, or VLPs, produced from HIV, rabies, VZV, SARS or Ebola - derived proteins, in accordance with the present invention do not comprise M1 protein. The M1 protein is known to bind RNA which may be considered a contaminant of a VLP preparation. The presence of RNA is undesired when obtaining regulatory approval for an antigenic (VLP) product, therefore a chimeric VLP preparation lacking RNA may be advantageous.

Although native HIV Env protein poorly accumulates in plants, a chimeric HIV Env protein, fused to a transmembrane (TM) and cytoplasmic tail (CT) domains from influenza HA accumulates at high level, and buds into HIV VLPs in absence of core or matrix protein, in plants.

### BRIEF DESCRIPTION OF THE DRAWINGS

These and other features of the invention will become more apparent from the following description in which reference is made to the appended drawings wherein:

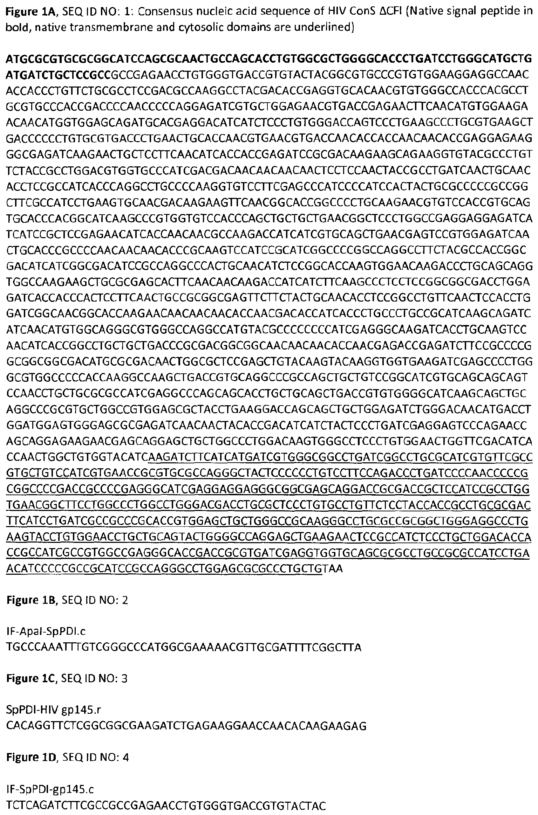

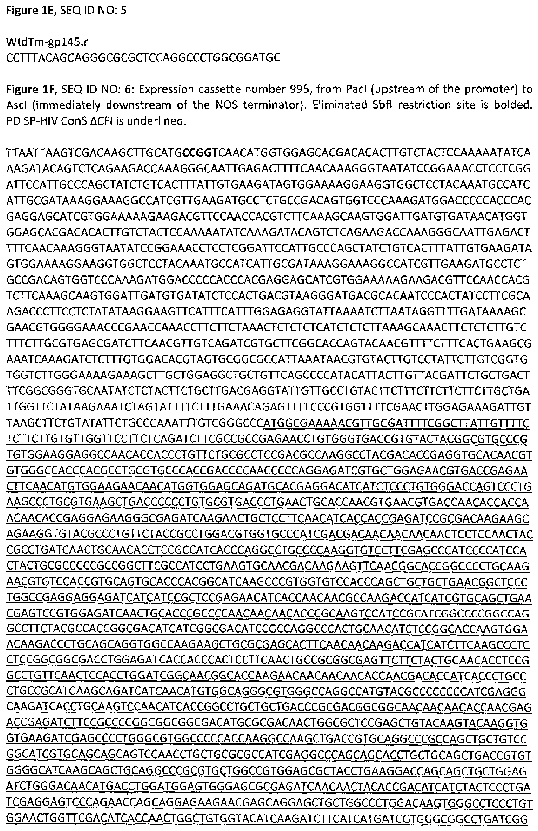

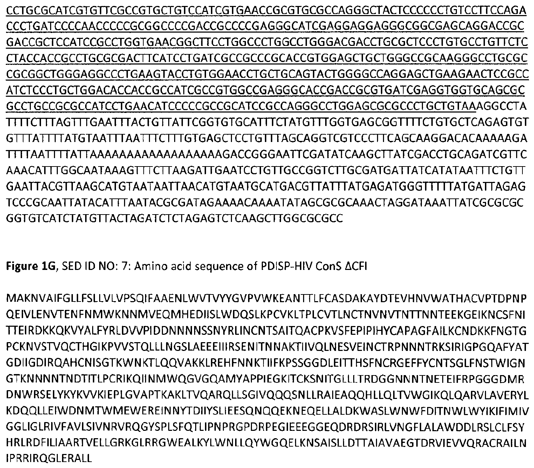

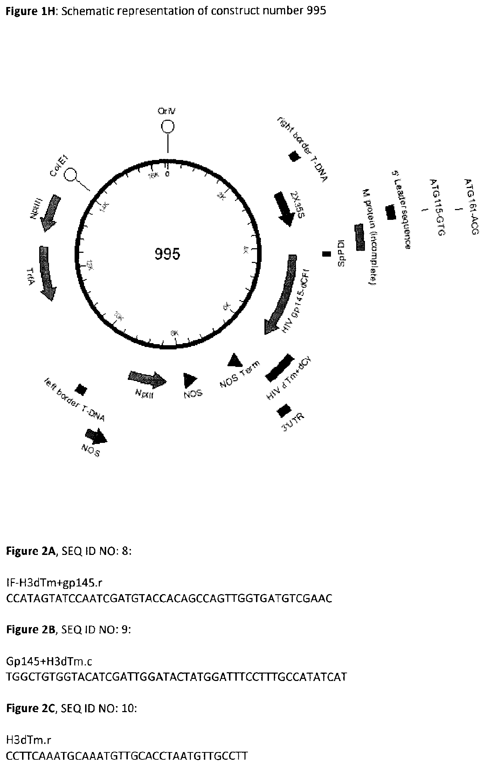

**Figure 1** shows several nucleotide and amino sequences, and expression cassettes for HIV in accordance with various embodiments of the present invention. **Figure 1A** shows a consensus nucleic acid sequence (SEQ ID NO: 1) of HIV ConS ΔCFI (Native signal peptide in bold, native transmembrane and cytosolic domains are underlined). **Figure 1B** shows a nucleotide sequence of oligonucleotide IF-ApaI-SpPDI.c (SEQ ID NO:2). **Figure 1C** shows a nucleotide sequence of oligonucleotide SpPDI-HIV gp145.r (SEQ ID NO:3). **Figure ID** shows a nucleotide sequence of oligonucleotide IF-SpPDI-gp145.c (SEQ ID NO:4). **Figure 1E** shows a nucleotide sequence of oligonucleotide WtdTm-gp145.r (SEQ ID NO:5). **Figure 1F** shows the nucleotide sequence (SEQ ID NO:6) of expression cassette number 995, from PacI (upstream of the promoter) to AscI (immediately downstream of the NOS terminator). Eliminated SbfI restriction site is bolded. PDISP-HIV ConS ΔCFI is underlined. **Figure 1G** shows an amino acid sequence of PDISP-HIV ConS ΔCFI (SEQ ID NO:7). **Figure 1H** shows a schematic representation of construct number 995.

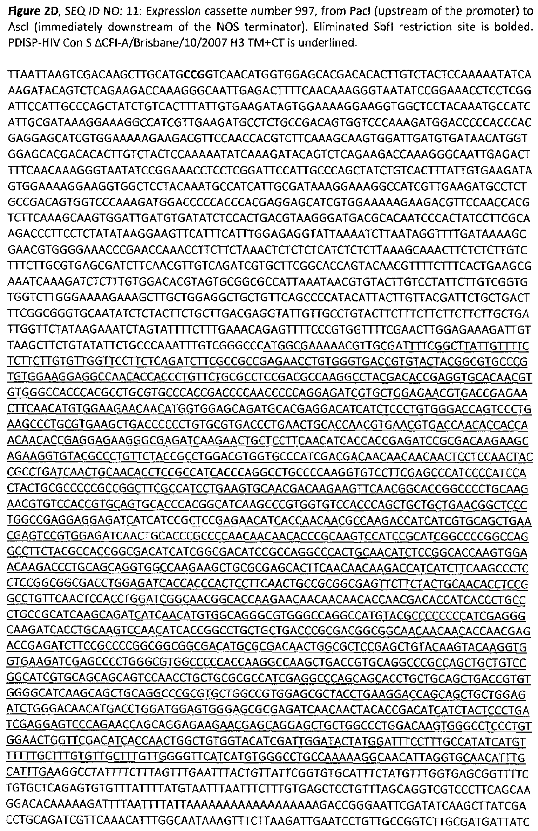

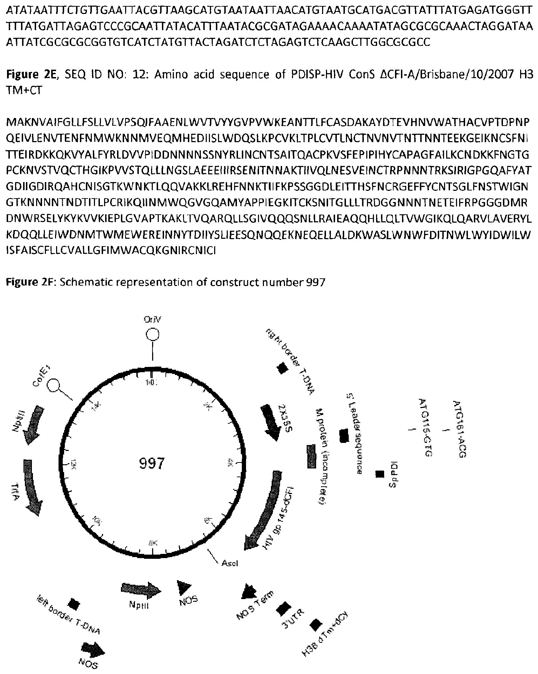

**Figure 2** shows several nucleotide and amino acid sequences, and expression cassettes for HIV in accordance with various embodiments of the present invention. **Figure 2A** shows a nucleotide sequence of oligonucleotide IF-H3dTm+gp145.r (SEQ ID NO:8). **Figure 2B** shows a nucleotide sequence of oligonucleotide Gp145+H3dTm.c (SEQ ID NO:9). **Figure 2C** shows a nucleotide sequence of oligonucleotide H3dTm.r (SEQ ID NO: 10). **Figure 2D** shows the nucleotide sequence (SEQ ID NO:11) of expression cassette number 997, from PacI (upstream of the promoter) to AscI (immediately downstream of the NOS terminator). Eliminated SbfI restriction site is bolded. PDISP-HIV Con S ΔCFI-A/Brisbane/10/2007 H3 TM+CT is underlined. **Figure 2E** shows an amino acid sequence of PDISP-HIV ConS ΔCFI-A/Brisbane/10/2007 H3 TM+CT (SEQ ID NO:12). **Figure 2F** shows a schematic representation of construct number 997.

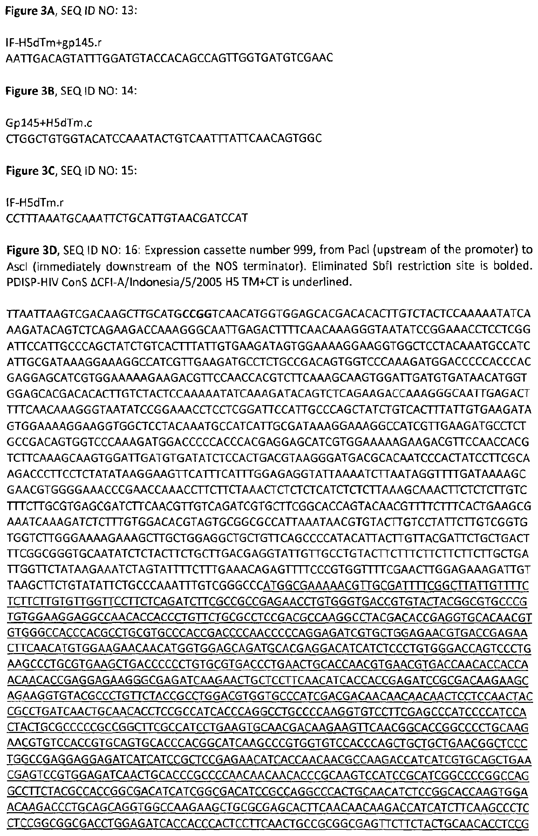

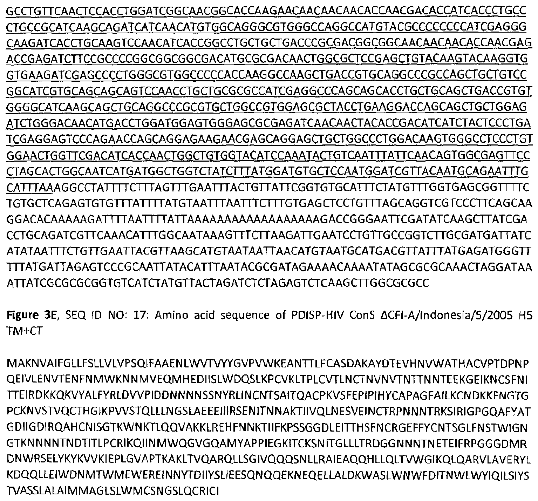

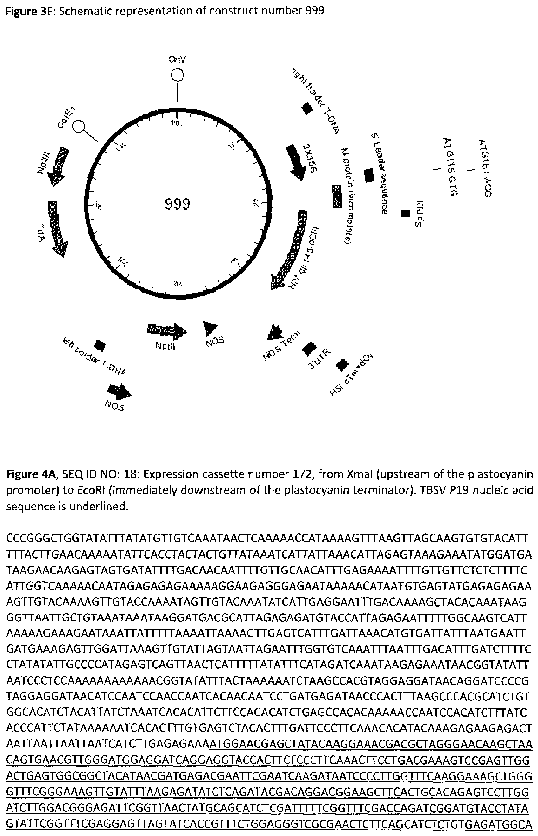

**Figure 3** shows several nucleotide and amino acid sequences, and expression cassettes for HIV in accordance with various embodiments of the present invention. **Figure 3A** shows a nucleotide sequence of oligonucleotide IF-H5dTm+gp145.r (SEQ ID NO: 13). **Figure 3B** shows a nucleotide sequence of oligonucleotide Gp145+H5dTm.c (SEQ ID NO:14). **Figure 3C** shows a nucleotide sequence of oligonucleotide IF-H5dTm.r (SEQ ID NO:15). **Figure 3D** shows the nucleotide sequence (SEQ ID NO:16) of expression cassette number 999, from PacI (upstream of the promoter) to AscI (immediately downstream of the NOS terminator). Eliminated SbfI restriction site is bolded. PDISP-HIV ConS ΔCFI-A/Indonesia/5/2005 H5 TM+CT is underlined. **Figure 3E** shows an amino acid sequence of PDISP-HIV ConS ΔCFI-A/Indonesia/5/2005 H5 TM+CT (SEQ ID NO: 17). **Figure 3F** shows a schematic representation of construct number 999.

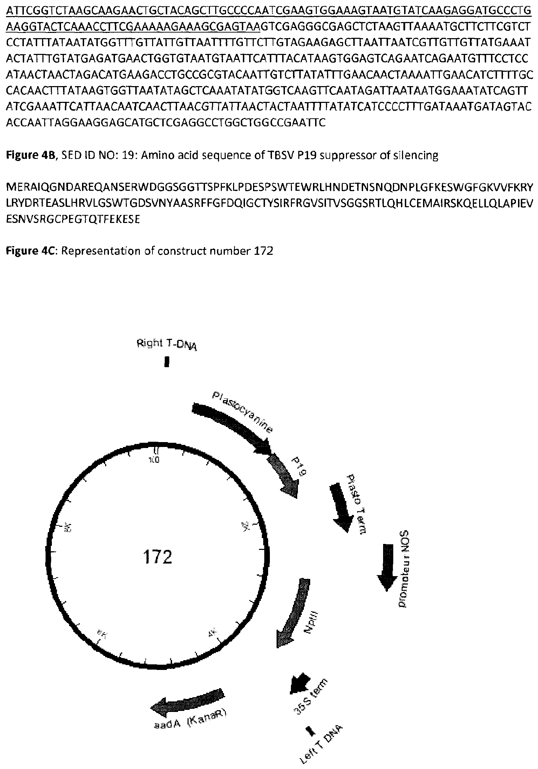

**Figure 4** shows an amino acid sequence and several expression cassettes for p19 in accordance with various embodiments of the present invention. **Figure 4A** shows the nucleotide sequence (SEQ ID NO: 18) of expression cassette number 172, from XmaI (upstream of the plastocyanin promoter) to EcoRI (immediately downstream of the plastocyanin terminator). TBSV P19 nucleic acid sequence is underlined. **Figure 4B** shows an amino acid sequence (SEQ ID NO:19) of TBSV P19 suppressor of silencing. **Figure 4C** shows a representation of construct number 172.

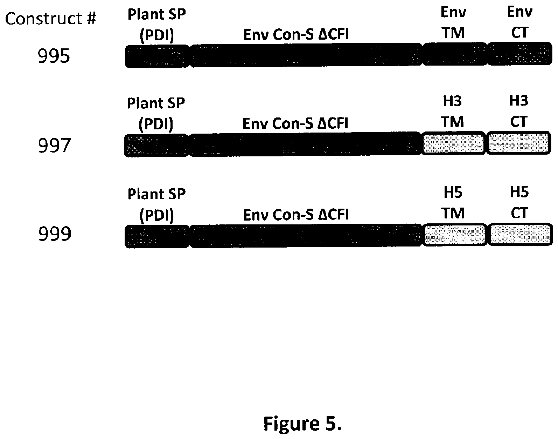

**Figure 5** shows a schematic representation of chimeric HIV Env genes expressed as described herein.

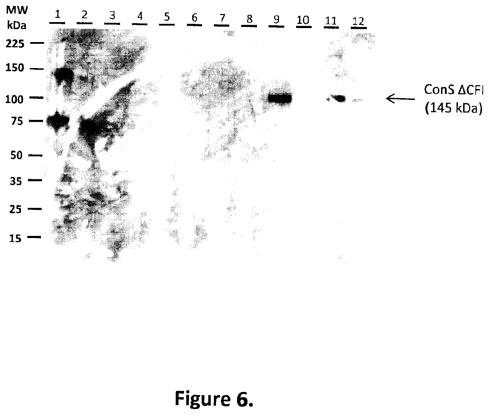

**Figure 6** shows a Western blot analysis of HIV Env protein expression in agroinfiltrated *Nicotiana benthamiana* leaves. Lanes 1 to 4, recombinant HIV-1 gp160 (ab68171), 100, 50, 10 and 5 ng, respectively, in 20 µg of leaf proteins extracted from mock-infiltrated plants (positive controls). Lane 5, 20 µg proteins form mock-infiltrated plants (negative control). Lanes 6 to 8, proteins extracted from AGL1/995-infiltrated leaves (20 µg, 10µg and 2 µg, respectively, completed to 20 µg with leaf proteins extracted from mock-infiltrated plants). Lanes 9 and 10, proteins extracted from AGL1/997-infiltrated leaves (20 µg and 10 µg, respectively, completed to 20 µg with leaf proteins extracted from mock-infiltrated plants). Lanes 11 and 12, proteins extracted from AGL1/999-infiltrated leaves (20 µg and 10 µg, respectively, completed to 20 µg with leaf proteins extracted from mock-infiltrated plants).

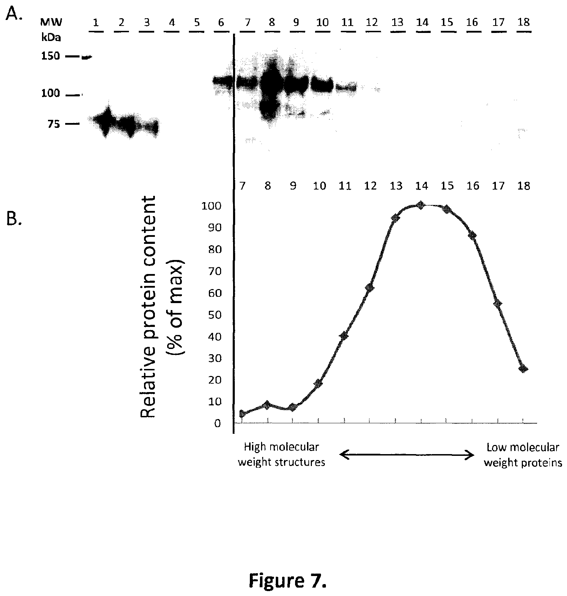

**Figure 7** shows a characterization of HIV ConS ΔCFI-derived structures by size exclusion chromatography. Protein extracts from leaves infiltrated with AGL1/999, producing Env/H5 chimeric protein, were separated by gel filtration on a calibrated S-500 high-resolution column. (A) The HIV Env protein content of elution fractions was revealed by immunodetection using anti-gp120 antibodies. Lanes 1 to 4, recombinant HIV-1 gp160 (ab68171), 100, 50, 10 and 5 ng, respectively, in 20 µg of leaf proteins extracted from mock-infiltrated plants (positive controls). Lane 5, 20 µg proteins form mock-infiltrated plants (negative control). Lane 6, 20 µg of proteins extracted from AGL1/999-infiltrated leaves. Lanes 7 to 18, elution fractions 7 to 18 from gel filtration chromatography. (B) Relative protein content of elution fractions 7 to 18 from the gel filtration chromatography.

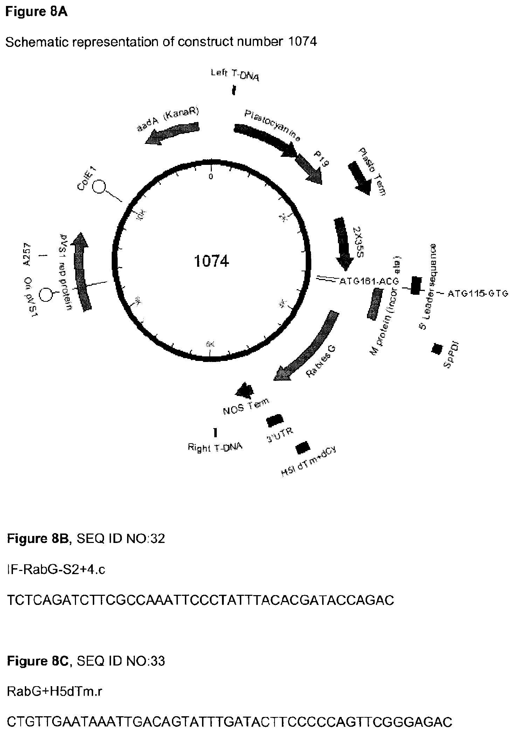

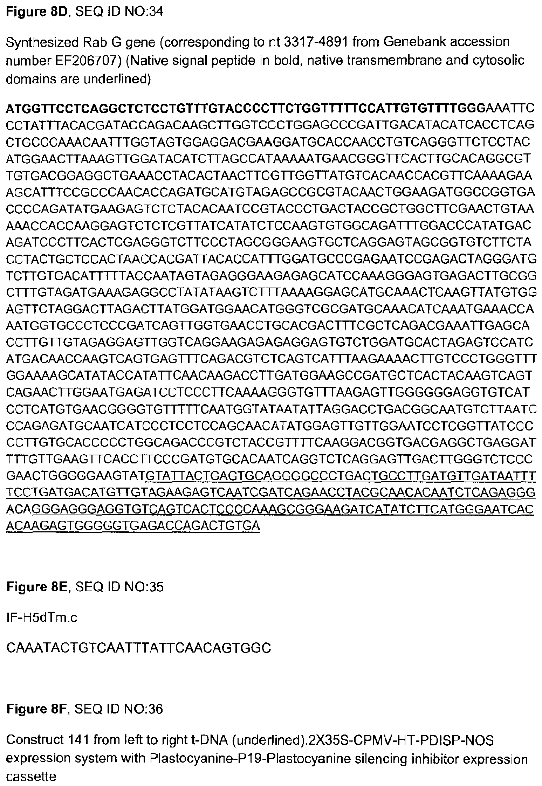

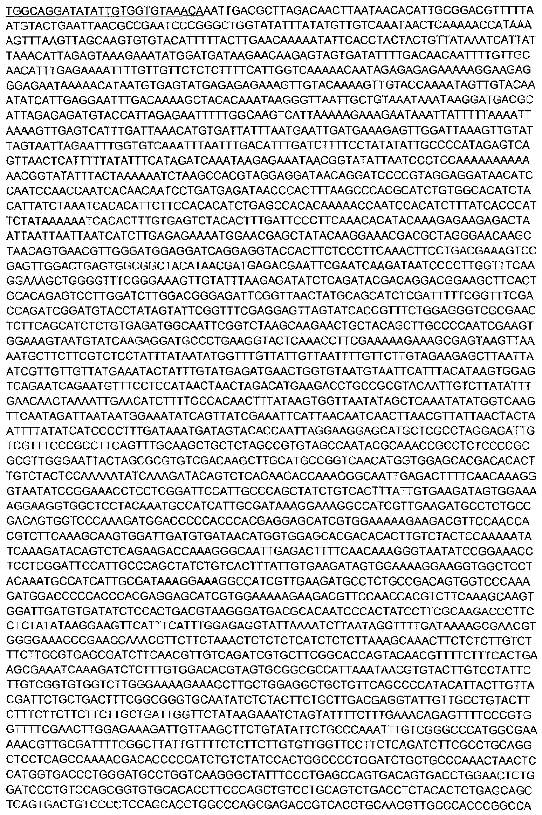

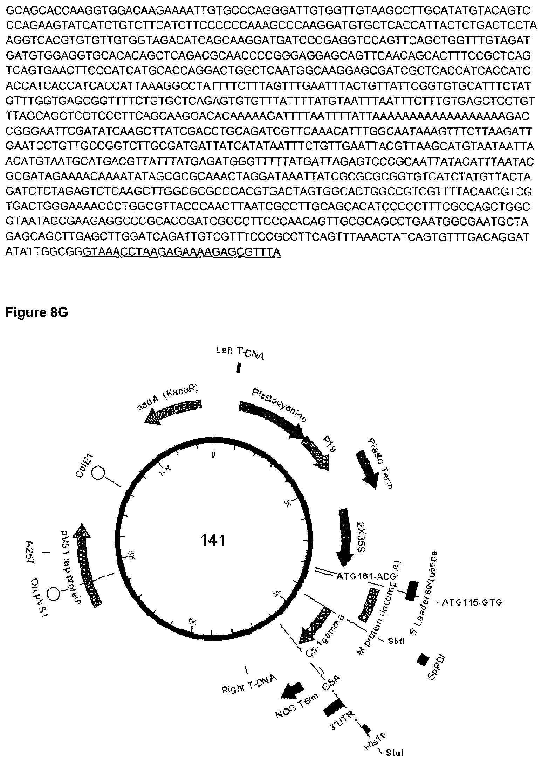

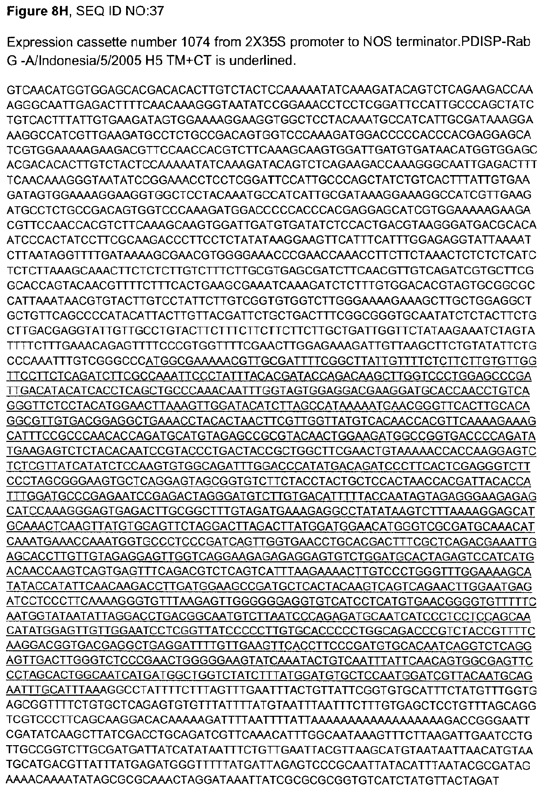

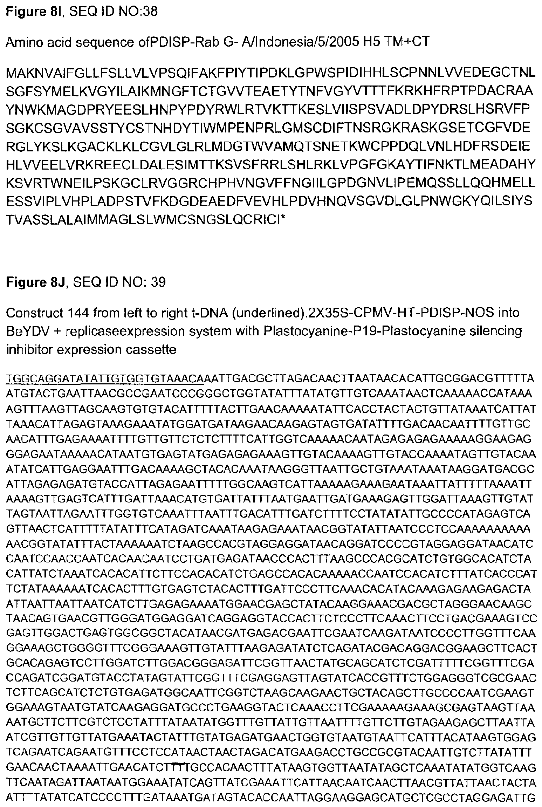

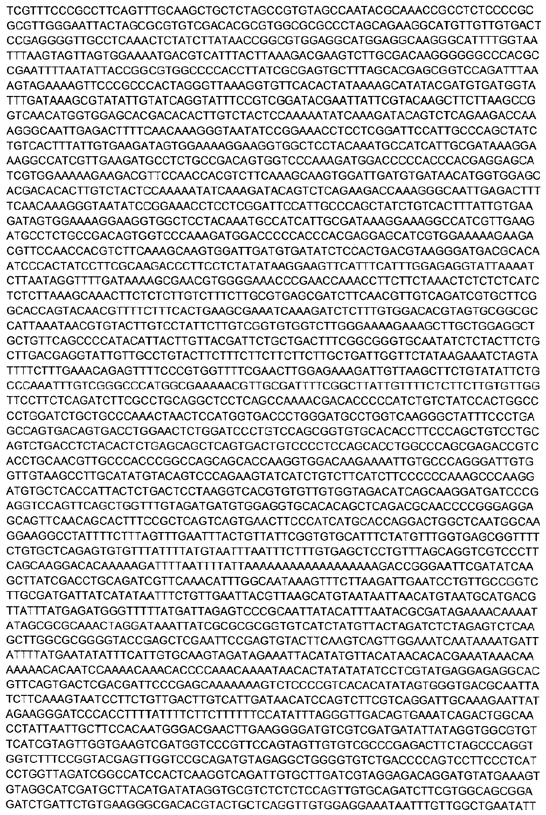

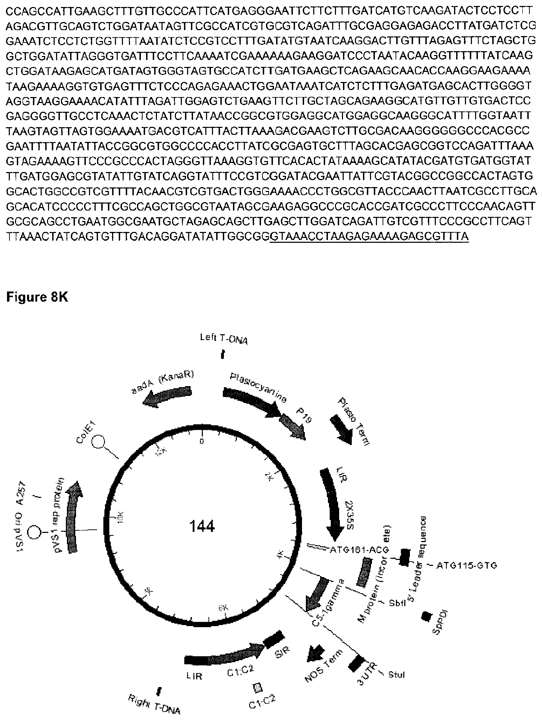

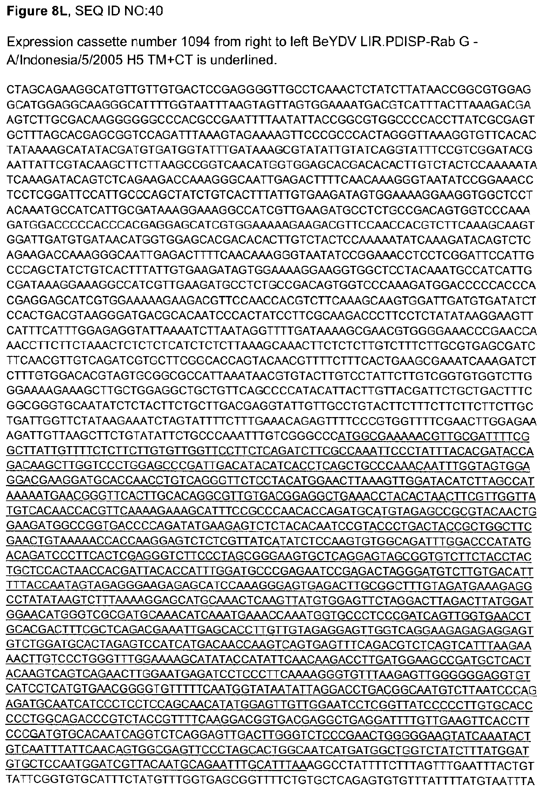

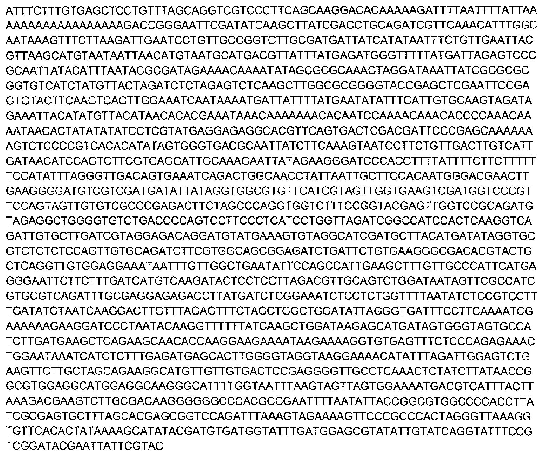

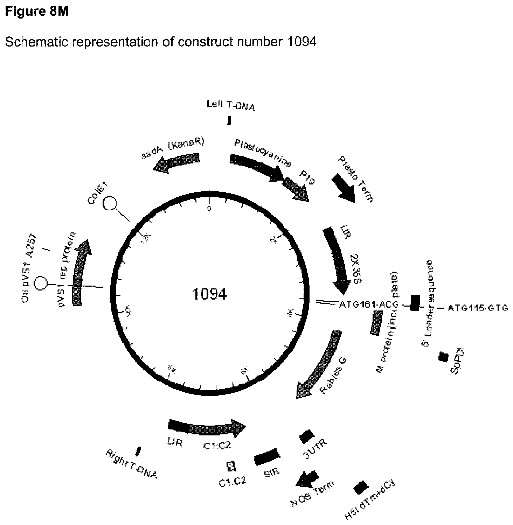

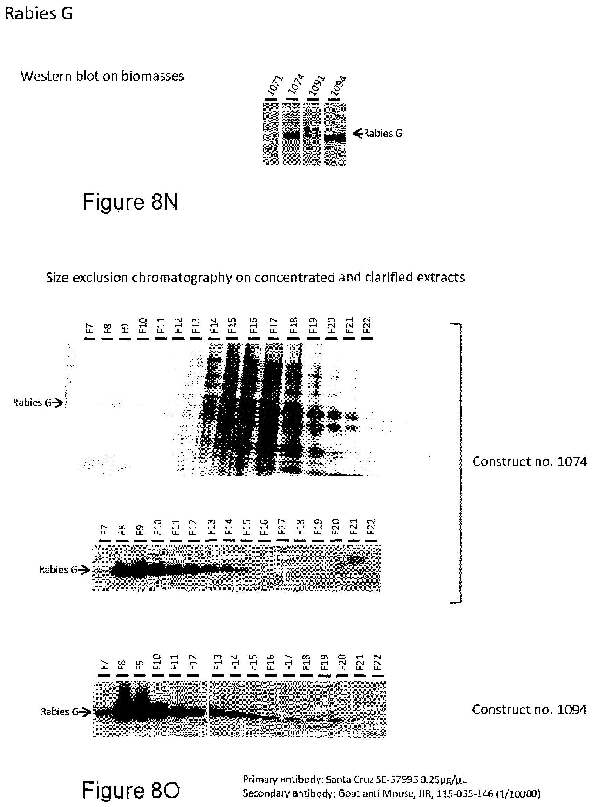

**Figure 8** shows several nucleotide and amino sequences, and expression cassettes for rabies in accordance with various embodiments of the present invention. **Figure 8A** shows a schematic of construct 1074 (C-2X35S-Rabies glycoprotein G (RabG)+H5 A/Indonesia/5/2005 transmembrane domain and cytoplasmic tail (TM+CT)-NOS on Plastocyanine-P19-Plastocyanine silencing inhibitor containing vector). **Figure 8B** shows primer IF-RabG-S2+4.c (SEQ ID NO:32). **Figure 8C** shows primer RabG+H5dTm.r (SEQ IDNO:33). **Figure 8D** shows synthesized Rab G gene (corresponding to nt 3317-4891 from Genebank accession number EF206707; native signal peptide in bold, native transmembrane and cytosolic domains are underlined; SEQ ID NO:34). **Figure 8E** shows primer IF-H5dTm.c (SEQ ID NO:35). **Figure 8F** shows construct 141 from left to right t-DNA (underlined).2X35S-CPMV-HT-PDISP-NOS expression system with Plastocyanine-P19-Plastocyanine silencing inhibitor expression cassette (SEQ ID NO:36). **Figure 8G** shows a schematic representation of construct 141.SbfI and StuI restriction enzyme sites used for plasmid linearization are annotated on the representation. **Figure 8H** shows the nucleotide sequence of expression cassette number 1074 from 2X35S promoter to NOS terminator.PDISP-Rab G -A/Indonesia/5/2005 H5 TM+CT is underlined (SEQ ID NO:37). **Figure 8I** shows the amino acid sequence ofPDISP-Rab G-A/Indonesia/5/2005 H5 TM+CT (SEQ ID NO:38). **Figure 8J** shows the nucleotide sequence for construct 144 from left to right t-DNA (underlined).2X35S-CPMV-HT-PDISP-NOS into BeYDV + replicaseexpression system with Plastocyanine-P19-Plastocyanine silencing inhibitor expression cassette (SEQ ID NO:39). **Figure 8K** shows a schematic representation of construct 144.SbfI and StuI restriction enzyme sites used for plasmid linearization are annotated on the representation. **Figure 8L** shows the nucleotide sequence of expression cassette number 1094 from right to left BeYDV LIR.PDISP-Rab G -A/Indonesia/5/2005 H5 TM+CT is underlined (SEQ ID NO:40). **Figure 8M** shows a schematic representation of construct number 1094. **Figure 8N** shows Immunoblot analysis of expression of rabies G protein in plant. The rabies G protein was expressed in fusion with PDI Sp (construct 1071), BeYDV + rep, H5A/ Indo TM+CT domain or a combination thereof. More specifically, construct 1074 is a fusion of rabies G protein with PDI Sp and H5A/ Indo TM+CT domain. Construct 1094 is a fusion of rabies G protein with BeYDV + rep, PDI Sp and H5A/ Indo TM+CT domain. Construct 1091 is a fusion of rabies G protein with PDI Sp and BeYDV + rep. **Figure 8O** shows Immunoblot analysis of size exclusion chromatography on concentrated and clarified extracts of protein expressed from construct 1074 and construct 1094.

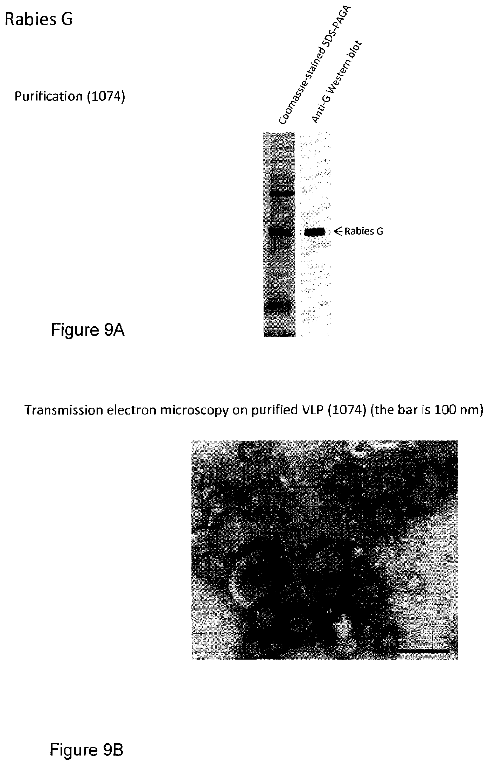

**Figure 9A** shows Immunoblot analysis of the purified rabies G protein expressed from construct 1074. Figure 9B shows a transmission electron microscopy picture of the purified VLP derived from expression of construct 1074.

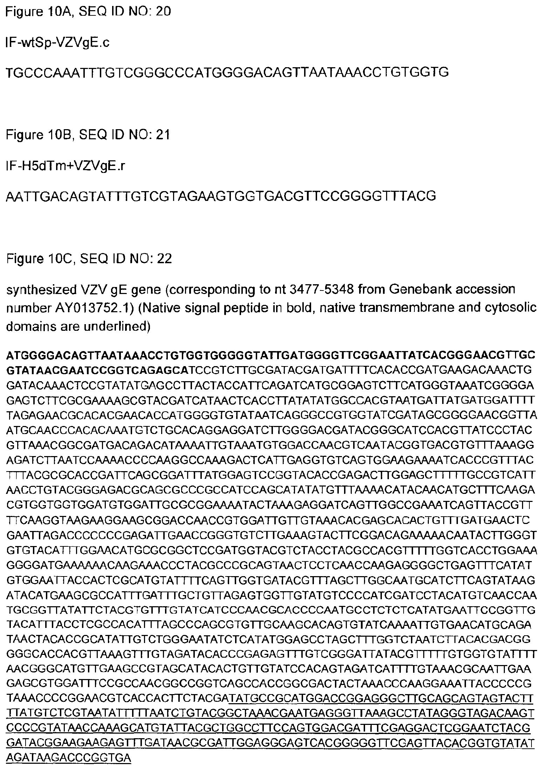

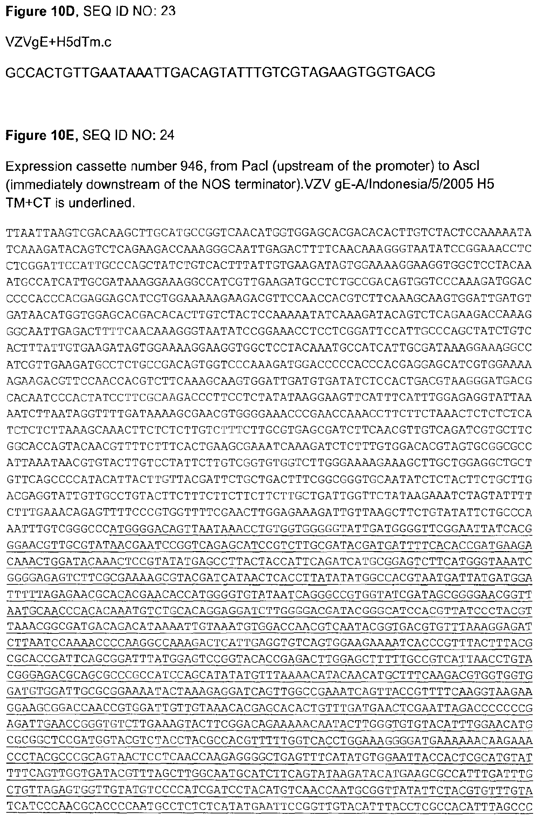

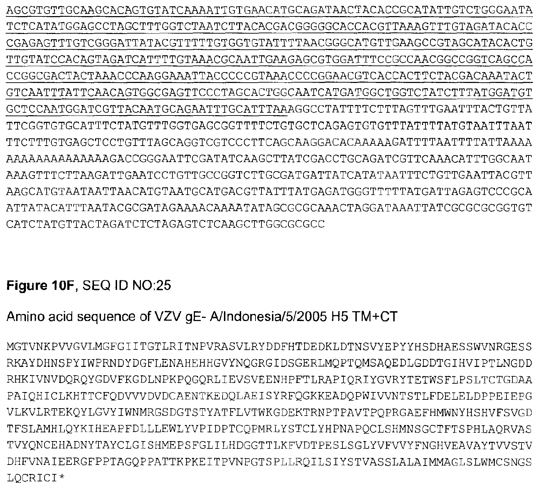

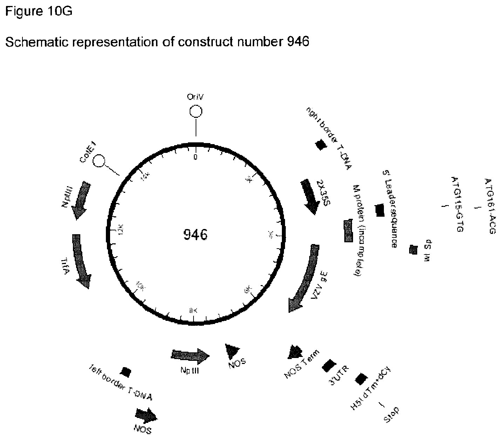

**Figure 10** shows sequences to prepare A-2X35S-Varicella Zoster Virus glycoprotein E (VZVgE)+H5 A/Indonesia/5/2005 transmembranedomain and cytoplasmictail (TM+CT)-NOS (Construct number 946). **Figure 10A** shows primer IF-wtSp-VZVgE.c (SEQ ID NO 20); **Figure 10B** shows primer IF-H5dTm+VZVgE.r (SEQ ID NO: 21). **Figure 10C** shows synthesized VZV gE gene (SEQ ID NO:22; corresponding to nt 3477-5348 from Genebank accession number AY013752.1) (Native signal peptide in bold, native transmembrane and cytosolic domains are underlined). **Figure 10D** shows primer for VZVgE+HSdTm.c (SEQ ID NO:23). **Figure 10E** shows expression cassette number 946 (SEQ ID NO:24), from PacI (upstream of the promoter) to AscI (immediately downstream of the NOS terminator).VZV gE-A/Indonesia/5/2005 H5 TM+CT underlined. **Figure 10F** shows amino acid sequence of VZV gE- A/Indonesia/5/2005 H5 TM+CT (SEQ ID NO:25). **Figure 10G** shows a schematic representation of construct number 946

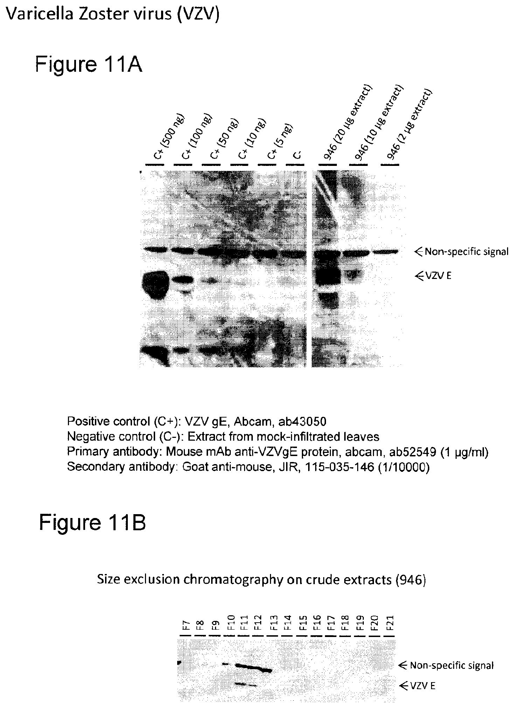

**Figure 11A** shows Immunoblot analysis of expression of Varicella Zoster Virus (VZV) E protein. Lanes 1 to 5, recombinant VZV gE, 500, 100, 50, 10 and 5 ng, respectively (positive controls). Lane 6 extract from mock-infiltrated leaves (negative control). Lanes 7-9 recombinant protein from construct 946, 20, 10 and 2 µg of extract respectively. Construct 946 comprises VZV gE gene with wild type signal peptide and H5A/ Indo TM+CT domain. **Figure 11B** shows Immunoblot analysis of expression of size exclusion chromatography on crude extracts (construct 946).

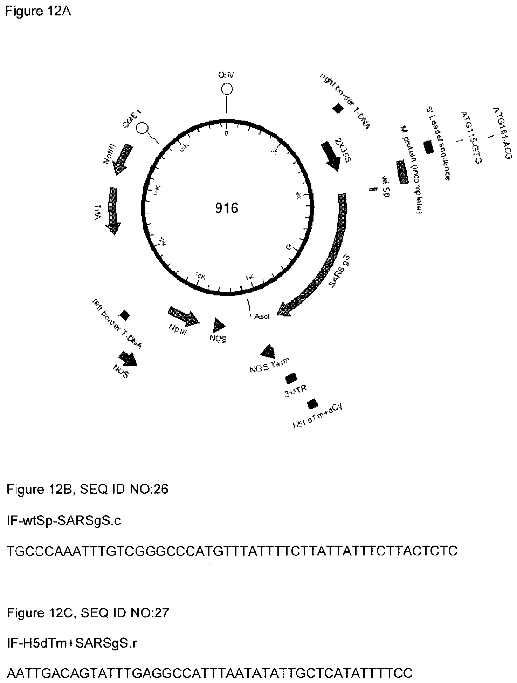

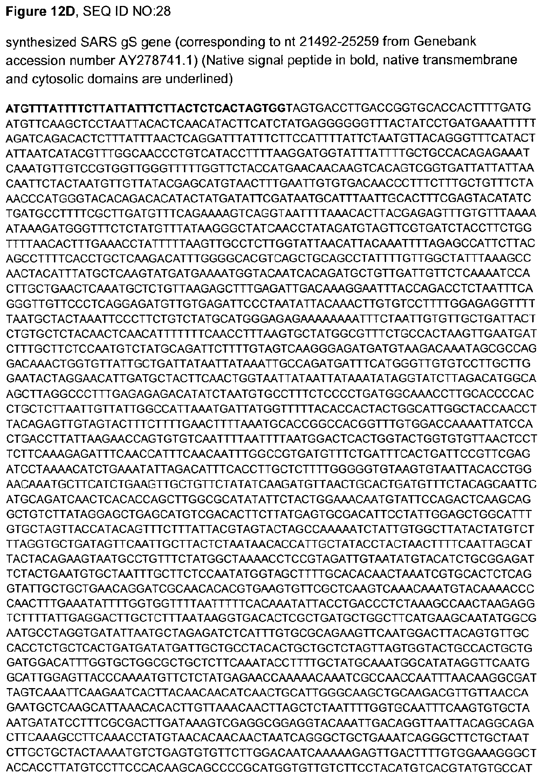

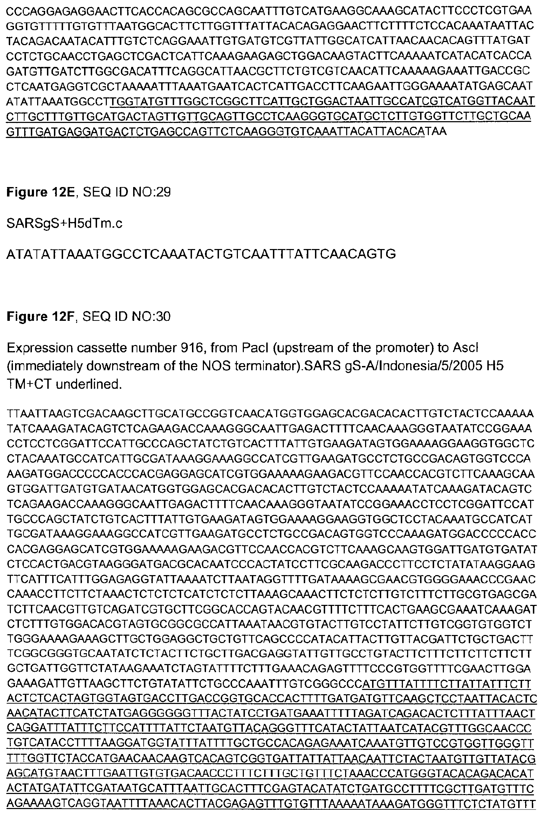

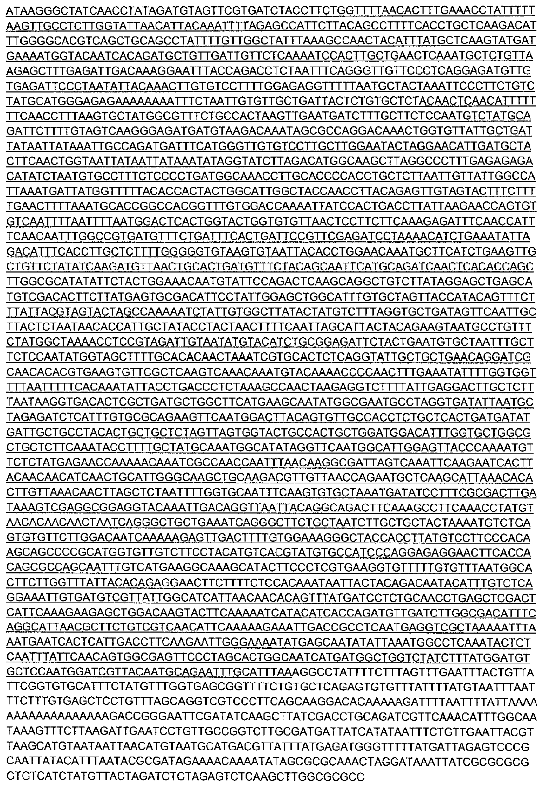

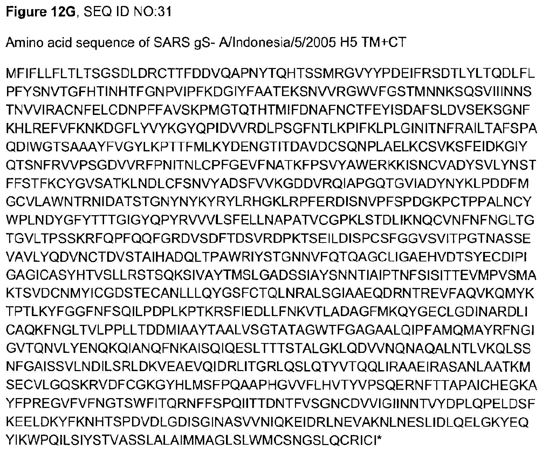

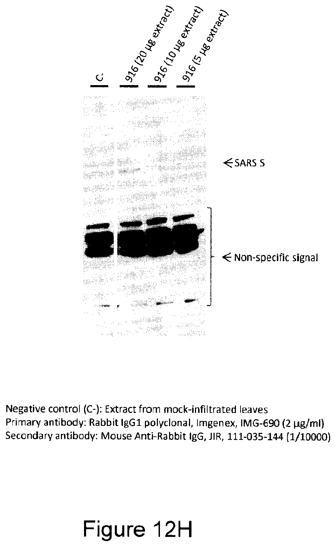

**Figure 12** shows several nucleotide and amino sequences, and expression cassettes for SARS in accordance with various embodiments of the present invention. **Figure 12A** shows a schematic of Construct number 916 (B-2X35S- Severe Acute Respiratory Syndrome Virus glycoprotein S (SARS gS)+H5 A/Indonesia/5/2005 transmembrane domain and cytoplasmic tail (TM+CT)-NOS). **Figure 12B** shows primer IF-wtSp-SARSgS.c (SEQ ID NO:26). **Figure 12C** shows primer IF-H5dTm+SARSgS.r (SEQ ID NO:27). **Figure 12D** shows synthesized SARS gS gene (SEQ ID NO:28; corresponding to nt 21492-25259 from Genebank accession number AY278741.1; native signal peptide in bold, native transmembrane and cytosolic domains are underlined). **Figure 12E** shows primer SARSgS+H5dTm.c SEQ ID NO:29). **Figure 12F** shows the nucleotide sequence of expression cassette number 916 (SEQ ID NO:30), from PacI (upstream of the promoter) to AscI (immediately downstream of the NOS terminator).SARS gS-A/Indonesia/5/2005 H5 TM+CT underlined. **Figure 12G** shows the amino acid sequence of SARS gS-A/Indonesia/5/2005 H5 TM+CT (SEQ ID NO:31). **Figure 12H** shows Immunoblot analysis of expression of Severe acute respiratory syndrome (SARS) virus protein S. Construct 916 comprises SARS gS gene with wild type signal peptide and H5 A/Indo transmembrane and cytosolic tail domain. Lane 1 extract from mock-infiltrated plants (negative controls). Lanes 2 to 4, recombinant protein from construct 916 (20, 10 and 5 µg of extract).

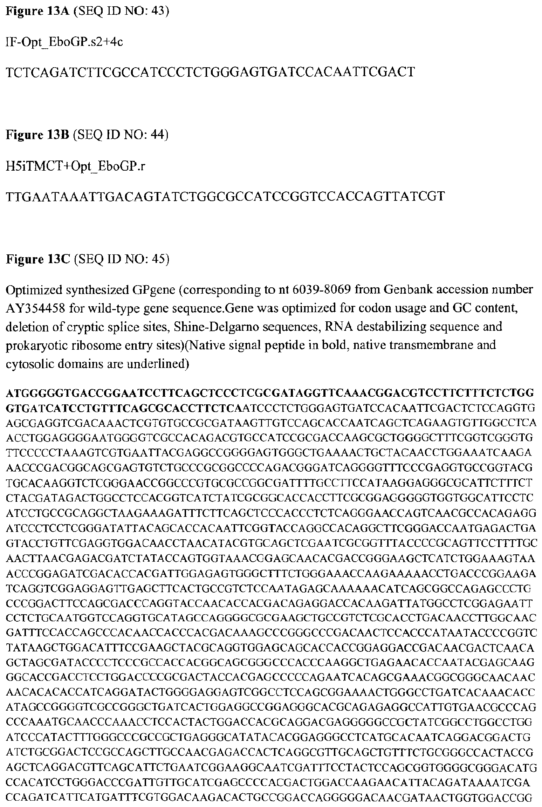

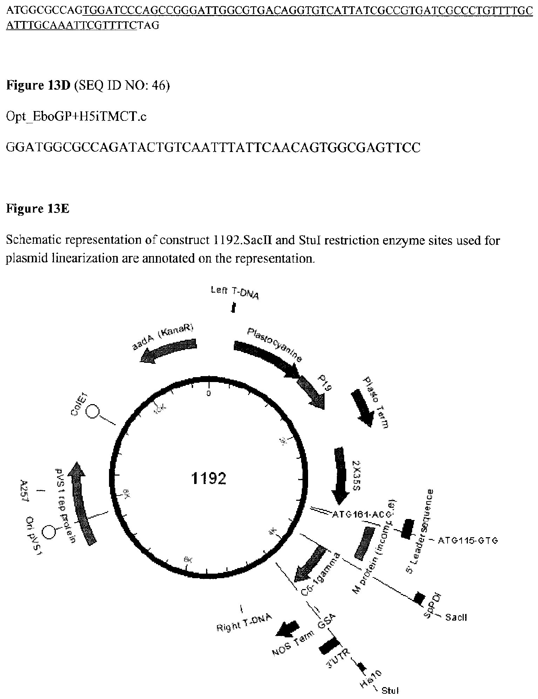

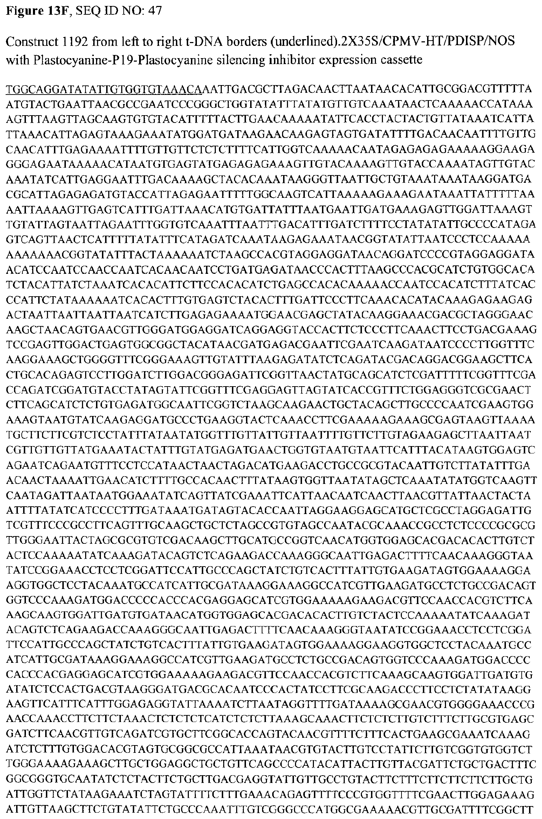

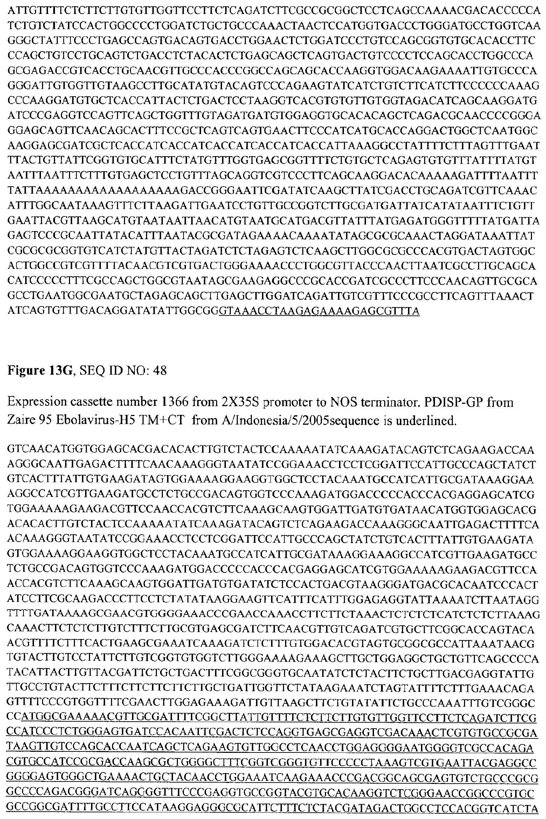

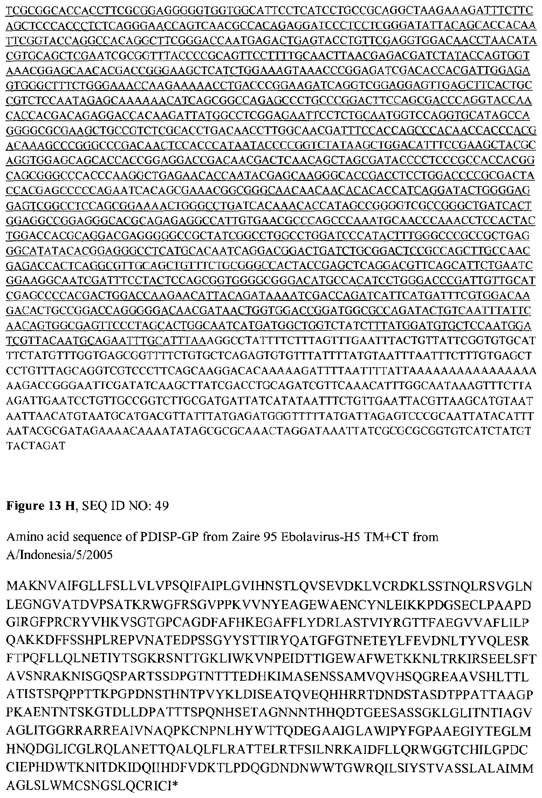

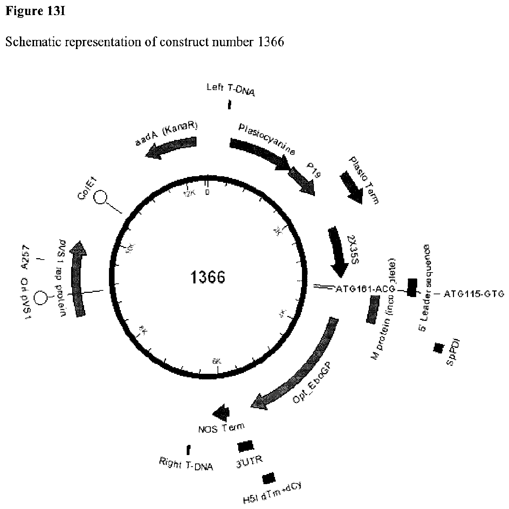

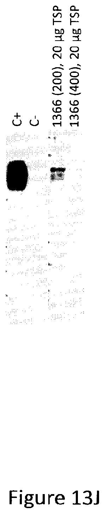

**Figure 13** shows several nucleotide and amino sequences, and expression cassettes for Ebola in accordance with various embodiments of the present invention. **Figure 13A** shows the nucleotide sequence of primer IF-Opt_EboGP.s2+4c (SEQ ID NO:43). **Figure 13B** shows the nucleotide sequence of primer H5iTMCT+Opt_EboGP.r (SEQ ID NO:44). **Figure 13C** shows the nucleotide sequence of optimized synthesized GPgene (corresponding to nt 6039-8069 from Genbank accession number AY354458 for wild-type gene sequence. The sequence (SEQ ID NO:45) was optimized for codon usage and GC content, deletion of cryptic splice sites, Shine-Delgarno sequences, RNA destabilizing sequence and prokaryotic ribosome entry sites; Native signal peptide in bold, native transmembrane and cytosolic domains are underlined). **Figure 13D** shows the nucleotide sequence of primer opt_EboGP+H5iTMCT.c (SEQ ID NO: 46). **Figure 13E** shows the schematic representation of construct 1192.SacII and StuI restriction enzyme sites used for plasmid linearization are annotated on the representation. **Figure 13F** shows the nucleotide sequence for construct 1192 from left to right t-DNA borders (underlined).2X35S/CPMV-HT/PDISP/NOS with Plastocyanine-P19-Plastocyanine silencing inhibitor expression cassette (SEQ ID NO. 47). **Figure 13G** shows the nucleotide sequence for expression cassette number 1366 from 2X35S promoter to NOS terminator.PDISP-GP from Zaire 95 Ebolavirus-H5 TM+CT from A/Indonesia/5/2005sequence is underlined (SEQ ID NO:48). **Figure 13H** shows the amino acid sequence of PDISP-GP from Zaire 95 Ebolavirus-H5 TM+CT from A/Indonesia/5/2005 9SEQ ID NO. 49). **Figure 13I** shows a schematic representation of construct number 1366. **Figure 13J** shows an immunoblot analysis of expression of Ebola virus glycoprotein (GP) protein. Five hundred nanograms were spiked in 20 µg of proteins extracted from mock-infiltrated plants and loaded as positive control (C+). Twenty micrograms of proteins extracted from mock-infiltrated plants were loaded as negative control (C-). Construct 1366 comprises the Ebola virus GP gene with wild type signal peptide and H5A/ Indo TM+CT domain. Numbers in parenthesis indicate the amount of initial bacterial culture that was used in the preparation of the inoculum for infiltration. (200) indicates that 200 ml of culture was mixed with 2,3 L of infiltration buffer and (400) indicates that 400 ml of culture was mixed with 2,1 L of infiltration buffer.

### DETAILED DESCRIPTION

The present invention relates to virus-like particles (VLPs). More specifically, the present invention is directed to VLPs comprising chimeric virus proteins, and methods of producing chimeric VLPs in plants. The VLPs comprise a fusion (chimeric) protein comprising, in series, an ectodomain from a virus trimeric surface protein (viral trimeric surface protein) fused to a transmembrane domain and cytosolic tail domain (TM/CT). The ectodomain from the virus trimeric surface protein is heterologous with respect to the TM/CT. The TM/CT is a TM/CT from an influenza hemagglutinin (HA).

The virus trimeric surface protein (also referred to as viral trimeric surface protein) is a protein found on the surface of an enveloped virus in the form of a trimer (usually homotrimer) and comprises a transmembrane domain and cytoplasmic tail domain (TM/CT) positioned at the C-terminal end of each monomer and an ectodomain positioned at the N-terminal region of each monomer. The virus trimeric surface protein may be a glycoprotein or an envelope protein. A trimer is a macromolecular complex formed by three, usually non-covalently bound proteins. Without wishing to be bound by theory, the trimerization domain of a protein may be important for the formation such trimers. Therefore monomers of the virus trimeric surface protein or fragment thereof may comprise a trimerization domain. The virus trimeric surface protein or fragment thereof further comprise an ectodomain. The ectodomain of the trimeric surface protein is exposed to the outer environment and does not include a transmembrane domain and cytoplasmic tail domain. As described herein, the ectodomain from the virus trimeric surface protein is not derived from an influenza virus. If a fragment of the virus trimeric surface protein is used as described herein, it is preferred that the virus trimeric surface protein retains the ability to form a trimer.

The transmembrane domain and the cytoplasmic tail (TM/CT) of the virus trimeric surface protein, the TM/CT of the influenza protein, or both, maybe readily identified using methods as are know by one of skill in the art, for example, by determining the degree of hydrophobicity of an amino acid sequence of the protein, for example using a transmembrane prediction program (e.g. Expert Protein Analysis System; ExPASy.org, operated by the Swiss Institute of Bioinformatics; or the Dense Alignment Surface Method, Cserzo M., et al. 1997, Prot. Eng. vol. 10, no. 6, 673-676; Lolkema J.S. 1998, FEMS Microbiol Rev. 22, no 4, 305-322), by determining the hydropathy profile of the amino acid sequence of the protein (e.g. Kyte-Doolittle Hydropathy Profile), by determining the three-dimensional protein structure and identifying the structure that is thermodynamically stable in a membrane (e.g. a single alpha helix, a stable complex of several transmembrane alpha helices, a transmembrane beta barrel, a beta-helix, or any other structure that is thermodynamically stable in a membrane). Once identified, the TM/CT region of the virus trimeric surface protein may be replaced with the transmembrane and cytoplasmic tail obtained from an influenza virus as described below.

The chimeric VLP according to various embodiments of the present invention comprises a viral trimeric surface protein from which the transmembrane domain and cytosolic tail domains (TM/CT) are replaced with TM/CT obtained from an influenza HA. The virus trimeric surface protein is heterologous with respect to the TM/CT. The virus trimeric surface protein, or fragment thereof, may be derived without limitations from viruses of the Retroviridae, Rhabdoviridae, Herpesviridae, Coronaviridae, Paramyxoviridae, Poxviridae or Filoviridae family. The virus trimeric surface protein may be derived for example from the genus Lentivirus, Lyssavirus, Varicellovirus, Coronavirus or Ebolavirus. The virus trimeric surface protein may be derived from for example, but not limited to, HIV, Rabies virus, VZV, RSV, SARS virus, Ebola virus, Measles, Mumps, Varicella, Cytomegalovirus, Ebola/Filovirus, Herpesvirus, Epstein-Barr virus or Smallpox. The virus trimeric surface protein may be for example a trimeric surface protein and in it native form may comprise a transmembrane domain/cytoplasmic tail, for example but not limited to:
a. F protein (paramyxoviridae : RSV, Measles, Mumps, Newcastle Disease)
b. S protein (coronaviridae : SARS);
c. env protein (retroviridae : HIV);
d. G protein (rhabdoviridae : rabies);
e. envelope glycoproteins such as E, B, C, I, H (herpesviridae : VZV, cytomegalovirus, herpesvirus, Epstein-barr virus);
f. GP glycoprotein (filoviridae : ebola, marburg);
g. hemagglutinin (poxviridae : variola virus, vaccinia virus).
Non-limiting examples of several virus trimeric surface protein that may be used according to the present invention are described in more detail below.

### HIV

The present invention provides VLPs comprising chimeric human immunodeficiency (HIV) protein, and methods of producing chimeric HIV VLPs, the chimeric HIV protein comprising a fusion protein with for example HIV Env protein and a portion of an influenza hemagglutinin (HA), such as the transmembrane domain and cytosolic tail domain (TM/CT).

The present invention provides a nucleic acid comprising a nucleotide sequence encoding a chimeric HIV protein operatively linked to a regulatory region active in a plant.

Furthermore, the present invention provides a method of producing chimeric HIV VLPs in a plant. The method involves introducing a nucleic acid encoding a chimeric HIV protein operatively linked to a regulatory region active in the plant, into the plant, or portion of the plant, and incubating the plant or portion of the plant under conditions that permit the expression of the nucleic acid, thereby producing the chimeric HIV VLPs.

The present invention further provides for a VLP comprising a chimeric HIV protein. The VLP may be produced by the method as provided by the present invention.

### Rabies Virus

The present invention also provides VLPs comprising chimeric rabies virus protein, and methods of producing chimeric rabies VLPs, the chimeric rabies virus protein comprising a fusion protein with for example rabies G protein and a portion of an influenza hemagglutinin (HA), such as the transmembrane domain and cytosolic tail domain (TM/CT).

The present invention provides a nucleic acid comprising a nucleotide sequence encoding a chimeric rabies virus protein operatively linked to a regulatory region active in a plant.

Furthermore, the present invention provides a method of producing chimeric rabies virus VLPs in a plant. The method involves introducing a nucleic acid encoding a chimeric rabies virus protein operatively linked to a regulatory region active in the plant, into the plant, or portion of the plant, and incubating the plant or portion of the plant under conditions that permit the expression of the nucleic acid, thereby producing the chimeric rabies virus VLPs.

The present invention further provides for a VLP comprising a chimeric rabies virus protein. The VLP may be produced by the method as provided by the present invention.

### VZV

The present invention is also directed to VLPs comprising chimeric Varicella Zoster Virus (VZV) protein, and methods of producing chimeric VZV VLPs, the chimeric VZV protein comprising a fusion protein with for example VZV glycoprotein E and a portion of an influenza hemagglutinin (HA), such as the transmembrane domain and cytosolic tail domain (TM/CT).

The present invention provides a nucleic acid comprising a nucleotide sequence encoding a chimeric VZV protein operatively linked to a regulatory region active in a plant.

Furthermore, the present invention provides a method of producing chimeric VZV VLPs in a plant. The method involves introducing a nucleic acid encoding a chimeric VZV protein operatively linked to a regulatory region active in the plant, into the plant, or portion of the plant, and incubating the plant or portion of the plant under conditions that permit the expression of the nucleic acid, thereby producing the chimeric VZV VLPs.

The present invention further provides for a VLP comprising a chimeric VZV protein. The VLP may be produced by the method as provided by the present invention.

### SARS

The present invention is also directed to VLPs comprising chimeric Severe acute respiratory syndrome (SARS) virus protein, and methods of producing chimeric SARS VLPs, the chimeric SARS protein comprising a fusion protein with for example SARS glycoprotein S and a portion of an influenza hemagglutinin (HA), such as the transmembrane domain and cytosolic tail domain (TM/CT).

The present invention provides a nucleic acid comprising a nucleotide sequence encoding a chimeric SARS protein operatively linked to a regulatory region active in a plant.

Furthermore, the present invention provides a method of producing chimeric SARS VLPs in a plant. The method involves introducing a nucleic acid encoding a chimeric SARS virus protein operatively linked to a regulatory region active in the plant, into the plant, or portion of the plant, and incubating the plant or portion of the plant under conditions that permit the expression of the nucleic acid, thereby producing the chimeric SARS VLPs.

The present invention further provides for a VLP comprising a chimeric SARS virus protein. The VLP may be produced by the method as provided by the present invention.

### Ebola

The present invention is also directed to VLPs comprising chimeric Ebola virus protein, and methods of producing chimeric Ebola VLPs, the chimeric Ebola virus protein comprising a fusion protein with for example Ebola virus envelope glycoprotein and a portion of an influenza hemagglutinin (HA), such as the transmembrane domain and cytosolic tail domain (TM/CT).

The present invention provides a nucleic acid comprising a nucleotide sequence encoding a chimeric ebola virus protein operatively linked to a regulatory region active in a plant.

Furthermore, the present invention provides a method of producing chimeric Ebola VLPs in a plant. The method involves introducing a nucleic acid encoding a chimeric Ebola virus protein operatively linked to a regulatory region active in the plant, into the plant, or portion of the plant, and incubating the plant or portion of the plant under conditions that permit the expression of the nucleic acid, thereby producing the chimeric Ebola virus VLPs.

The present invention further provides for a VLP comprising a chimeric Ebola virus protein. The VLP may be produced by the method as provided by the present invention.

### Chimerization and VLP formation

Both vesicular stomatitis virus (a rhabdovirus like rabies) and Herpes Simplex virus (a herpesvirus like varicella-zoster virus) bud in a VSP4-dependent manner (Taylor et al. J. Virol 81:13631-13639, 2007; Crump et al., J. Virol 81:7380-7387, 2007). Since VSP4 interacts with the late domain of the matrix protein, this suggests that the matrix protein is required for budding and, as a corollary, for VLP production. However, as described herein, rabies and VZV VLPs can be produced without matrix protein when replacing the TM/CT domain with those of influenza HA. Without wishing to be bound by theory, this suggests that chimerization may eliminate the dependence on matrix protein co-expression for VLP formation for rabies and VZV.

The ectodomain of the viral trimeric surface protein as described above is fused to a transmembrane domain and cytosolic tail domain (TM/CT), so that the virus trimeric surface protein is heterologous with respect to the TM/CT. The TM/CT may be a TM/CT from a influenza hemagglutinin (HA), for example the TM/CT from H5 or H3, for example but not limited to A/Indonesia/5/05 sub-type (H5N1; "H5/Indo"; GenBank Accession No. ABW06108.1), H5 A/Vietnam/1194/2004(A-Vietnam; GenBank Accession No. ACR48874.1), H5 A/Anhui/1/2005 (A-Anhui; GenBank Accession No. ABD28180.1); H3 A/Brisbane/10/2007 ("H3/Bri"; GenBank Accession No. ACI26318.1), H3 A/Wisconsin/67/2005(A-WCN; GenBank Accession No. ABO37599.1). The TM/CT of boundaries of several H3 and H5 sequences are described in WO 2010/148511 For example, which is not to be considered limiting the amino acid sequence for TM/CT may include:
H5 (A/Indonesia/05/2005) TM/CT (SEQ ID NO:41): QILSIYSTVASSLALAIMMAGLSLWMCSNGSLQCRICI
H3 (A/Brisbane/10/2007) TM/CT (SEQ ID NO:42): DWILWISFAISCFLLCVALLGFIMWACQKGNIRCNICI
and any nucleotide sequences encoding the amino acid sequence of SEQ ID NO:41 or 42.

A specific nucleic acid sequence referred to in the present invention, may be "substantially homologous" or "substantially similar" to a sequence, or a sequence or a compliment of the sequence that hybridise to one or more than one nucleotide sequences as defined herein under stringent hybridisation conditions. Sequences are "substantially homologous" or "substantially similar" when at least about 70%, or more preferably 75% of the nucleotides match over a defined length of the nucleotide sequence providing that such homologous sequences exhibit one or more than one of the properties of the sequence, or the encoded product as described herein. For example a substantially homologous ectodomain from a virus trimeric surface protein, fused to a transmembrane domain and cytosolic tail domain obtained from H3 or H5, a transmembrane domain and cytosolic tail that is substantially homologous to the TM/CT of H3 or H5 and fused to ectodomain from a virus trimeric surface protein, or both a substantially homologous TM/CT and a substantially homologous ectodomain from a virus trimeric surface protein, form a VLP. Correct folding of the chimeric protein may be important for stability of the protein, formation of multimers, formation of VLPs and function. Folding of a protein may be influenced by one or more factors, including, but not limited to, the sequence of the protein, the relative abundance of the protein, the degree of intracellular crowding, the availability of cofactors that may bind or be transiently associated with the folded, partially folded or unfolded protein.

Such a sequence similarity may be determined using a nucleotide sequence comparison program, such as that provided within DNASIS (using, for example but not limited to, the following parameters: GAP penalty 5, #of top diagonals 5, fixed GAP penalty 10, k-tuple 2, floating gap 10, and window size 5). However, other methods of alignment of sequences for comparison are well-known in the art for example the algorithms of Smith & Waterman (1981, Adv. Appl. Math. 2:482), Needleman & Wunsch (J. Mol. Biol. 48:443, 1970), Pearson & Lipman (1988, Proc. Nat'l. Acad. Sci. USA 85:2444), and by computerized implementations of these algorithms (GAP, BESTFIT, FASTA, and BLAST, available through the NIH.), or by manual alignment and visual inspection (see, e.g., Current Protocols in Molecular Biology, Ausubel et al., eds. 1995 supplement), or using Southern or Northern hybridization under stringent conditions (see Maniatis et al., in Molecular Cloning (A Laboratory Manual), Cold Spring Harbor Laboratory, 1982). Preferably, sequences that are substantially homologous exhibit at least about 80% and most preferably at least about 90% sequence similarity over a defined length of the molecule.

An example of one such stringent hybridization conditions may be overnight (from about 16-20 hours) hybridization in 4 X SSC at 65°C, followed by washing in 0.1 X SSC at 65°C for an hour, or 2 washes in 0.1 X SSC at 65°C each for 20 or 30 minutes. Alternatively an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65°C each for 20 or 30 minutes, or overnight (16-20 hours), or hybridization in Church aqueous phosphate buffer (7% SDS; 0.5M NaPO₄ buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each for unique sequence regions.

A nucleic acid encoding a chimeric polypeptide, a chimeric protein, a fusion protein, a chimeric viral protein, or chimeric virus trimeric surface protein may be described as a "chimeric nucleic acid", or a "chimeric nucleotide sequence". For example, which is not to be considered limiting, a virus-like particle comprising a chimeric HIV protein, chimeric rabies virus protein, chimeric VZV protein, chimeric SARS or chimeric Ebola virus protein may be described as a "chimeric VLP".

By "chimeric protein", or "chimeric polypeptide", also referred to as a fusion protein, it is meant a protein or polypeptide that comprises amino acid sequences from two or more than two sources, for example but not limited to an ectodomain from a virus trimeric surface protein or fragment thereof, for example a F protein (e.g. RSV, Measles, Mumps, Newcastle Disease), S protein (e.g. SARS), env protein (HIV), G protein (rabies), envelope glycoproteins such as E, B, C, I, H (VZV, cytomegalovirus, herpesvirus, Epstein-barr virus), GP glycoprotein (e.g. ebola, marburg), hemagglutinin (e.g. variola virus, vaccinia virus), and for example the TM/CT of HA, that are fused as a single polypeptide. The chimeric protein or polypeptide may include a signal peptide that is the same as, or heterologous with, the remainder of the polypeptide or protein.

The term "signal peptide" is well known in the art and refers generally to a short (about 5-30 amino acids) sequence of amino acids, found generally at the N-terminus of a polypeptide that may direct translocation of the newly-translated polypeptide to a particular organelle, or aid in positioning of specific domains of the polypeptide chain relative to others. As a non-limiting example, the signal peptide may target the translocation of the protein into the endoplasmic reticulum and/or aid in positioning of the N-terminus proximal domain relative to a membrane-anchor domain of the nascent polypeptide to aid in cleavage and folding of the mature protein, for example which is not to be considered limiting, a mature HA protein.

A signal peptide (SP) may be native to the protein or virus protein, or a signal peptide may be heterologous with respect to the primary sequence of the protein or virus protein being expressed. A protein or virus protein may comprise a signal peptide from a first influenza type, subtype or strain with the balance of the HA from one or more than one different influenza type, subtype or strain. For example the native signal peptide of HA subtypes B H1, H2, H3, H5, H6, H7, H9 or influenza type B may be used to express the chimeric virus protein in a plant system. In some embodiments of the invention, the SP may be of an influenza type B, H1, H3 or H5; or of the subtype H1/Bri, H1/NC, H5/Indo, H3/Bri or B/Flo. In some embodiment the SP may be of HIV Env protein, rabies G protein, VZV glycoprotein E, SARS glycoprotein S or Eboloa virus envelope glycoprotein.

A signal peptide may also be non-native, for example, from a protein, viral protein, a virus trimeric surface protein or hemagglutinin of a virus other than the virus trimeric surface protein, or from a plant, animal or bacterial polypeptide. A non limiting exemple of a signal peptide that may be used is that of alfalfa protein disulfide isomerase (PDI SP; nucleotides 32-103 of Accession No. Z11499).

The present invention therefore provides for a chimeric virus protein comprising a native, or a non-native signal peptide, and nucleic acids encoding such chimeric virus proteins.

Correct folding of the expressed chimeric virus protein may be important for stability of the protein, formation of multimers, formation of VLPs, function of the chimeric virus protein and recognition of the chimeric virus protein by an antibody, among other characteristics. Folding and accumulation of a protein may be influenced by one or more factors, including, but not limited to, the sequence of the protein, the relative abundance of the protein, the degree of intracellular crowding, the pH in a cell compartment, the availability of cofactors that may bind or be transiently associated with the folded, partially folded or unfolded protein, the presence of one or more chaperone proteins, or the like.

Heat shock proteins (Hsp) or stress proteins are examples of chaperone proteins, which may participate in various cellular processes including protein synthesis, intracellular trafficking, prevention of misfolding, prevention of protein aggregation, assembly and disassembly of protein complexes, protein folding, and protein disaggregation. Examples of such chaperone proteins include, but are not limited to, Hsp60, Hsp65, Hsp 70, Hsp90, Hsp100, Hsp20-30, Hsp10, Hsp100-200, Hsp100, Hsp90, Lon, TF55, FKBPs, cyclophilins, ClpP, GrpE, ubiquitin, calnexin, and protein disulfide isomerases (see, for example, Macario, A.J.L., Cold Spring Harbor Laboratory Res. 25:59-70. 1995; Parsell, D.A. & Lindquist, S. Ann. Rev. Genet. 27:437-496 (1993); U.S. Patent No. 5,232,833). As described herein, chaperone proteins, for example but not limited to Hsp40 and Hsp70 may be used to ensure folding of a chimeric virus protein.

Examples of Hsp70 include Hsp72 and Hsc73 from mammalian cells, DnaK from bacteria, particularly mycobacteria such as *Mycobacterium* leprae, *Mycobacterium tuberculosis,* and *Mycobacterium bovis* (such as Bacille-Calmette Guerin: referred to herein as Hsp71). DnaK from *Escherichia coli,* yeast and other prokaryotes, and BiP and Grp78 from eukaryotes, such as A. *thaliana* (Lin et al. 2001 (Cell Stress and Chaperones 6:201-208). A particular example of an Hsp70 is A. *thaliana* Hsp70 (encoded by Genbank ref: AY120747.1). Hsp70 is capable of specifically binding ATP as well as unfolded polypeptides and peptides, thereby participating in protein folding and unfolding as well as in the assembly and disassembly of protein complexes.

Examples of Hsp40 include DnaJ from prokaryotes such as *E. coli* and mycobacteria and HSJ1, HDJ1 and Hsp40 from eukaryotes, such as alfalfa (Frugis et al., 1999. Plant Molecular Biology 40:397-408). A particular example of an Hsp40 is *M. sativa* MsJ1 (Genbank ref: AJ000995.1). Hsp40 plays a role as a molecular chaperone in protein folding, thermotolerance and DNA replication, among other cellular activities.

Among Hsps, Hsp70 and its co-chaperone, Hsp40, are involved in the stabilization of translating and newly synthesized polypeptides before the synthesis is complete. Without wishing to be bound by theory, Hsp40 binds to the hydrophobic patches of unfolded (nascent or newly transferred) polypeptides, thus facilitating the interaction of Hsp70-ATP complex with the polypeptide. ATP hydrolysis leads to the formation of a stable complex between the polypeptide, Hsp70 and ADP, and release of Hsp40. The association of Hsp70-ADP complex with the hydrophobic patches of the polypeptide prevents their interaction with other hydrophobic patches, preventing the incorrect folding and the formation of aggregates with other proteins (reviewed in Hartl, FU. 1996. Nature 381:571-579).

Native chaperone proteins may be able to facilitate correct folding of low levels of recombinant protein, but as the expression levels increase, the abundance of native chaperones may become a limiting factor. High levels of expression of chimeric virus protein in the agroinfiltrated leaves may lead to the accumulation of chimeric virus protein in the cytosol, and co-expression of one or more than one chaperone proteins such as Hsp70, Hsp40 or both Hsp70 and Hsp40 may reduce the level of misfolded or aggregated proteins, and increase the number of proteins exhibiting tertiary and quaternary structural characteristics that allow for formation of virus-like particles.

Therefore, the present invention also provides for a method of producing chimeric virus protein VLPs in a plant, wherein a first nucleic acid encoding a chimeric virus protein is co-expressed with a second nucleic acid encoding a chaperone. The first and second nucleic acids may be introduced to the plant in the same step, or may be introduced to the plant sequentially.

Chimeric VLPs produced from virus derived proteins, in accordance with the present invention do not comprise viral matrix or core protein. A viral matrix protein is a protein that organizes and maintains virion structure. Viral matrix proteins usually interact directly with cellular membranes and can be involved in the budding process. Viral core proteins are proteins that make up part of the nucelocapsid and typically are directly associated with the viral nucleic acid. Examples of viral matrix or core protein are influenza M1, RSV M and retrovirus gag proteins. The M1 protein is known to bind RNA (Wakefield L., and Brownlee G.G., Nucl Acids res 11:8569-8580, 1989) which is a contaminant of VLP preparation. The presence of RNA is undesired when obtaining regulatory approval for the chimeric VLP product, therefore a chimeric VLP preparation lacking RNA may be advantageous.

The use of the terms "regulatory region", "regulatory element" or "promoter" in the present application is meant to reflect a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" may includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, may also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.

In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element comprises a basal promoter element, responsible for the initiation of transcription, as well as other regulatory elements (as listed above) that modify gene expression.

There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et aI., 1994, Plant Cell 14: 125-130). An example of a leaf-specific promoter includes the plastocyanin promoter (see US 7,125,978.

An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, LR.P., 1998, Trends Plant Sci. 3, 352-358; Examples, of potential inducible promoters include, but not limited to, tetracycline-inducible promoter (Gatz, C.,1997, Ann. Rev. Plant Physiol. Plant Mol. BioI. 48,89-108; steroid inducible promoter (Aoyama. T. and Chua, N.H.,1997, Plant 1. 2, 397-404; and ethanol-inducible promoter (Salter, M.G., et aI, 1998, Plant 10urnal 16, 127-132; Caddick, M.X., et al,1998, Nature Biotech. 16, 177-180, cytokinin inducible IB6 and CKI 1 genes (Brandstatter, I. and K.ieber, 1.1.,1998, Plant Cell 10, 1009-1019; Kakimoto, T., 1996, Science 274,982-985; and the auxin inducible element, DR5 (Ulmasov, T., et aI., 1997, Plant Cell 9, 1963-1971;).

A constitutive regulatory region directs the expression of a gene throughout the various parts of a plant and continuously throughout plant development. Examples of known constitutive regulatory elements include promoters associated with the CaMV 35S transcript (Odell et aI., 1985, Nature, 313: 810-812), the rice actin 1 (Zhang et aI, 1991, Plant Cell, 3: 1155-1165), actin 2 (An et al., 1996, Plant J., 10: 107-121), or tms 2 (U.S. 5,428,147, and triosephosphate isomerase 1 (Xu et. aI., 1994, Plant Physiol. 106: 459-467) genes, the maize ubiquitin 1 gene (Cornejo et ai, 1993, Plant Mol. BioI. 29: 637-646), the *Arabidopsis* ubiquitin 1 and 6 genes (Holtorf et aI, 1995, Plant Mol. BioI. 29: 637-646), and the tobacco translational initiation factor 4A gene (Mandel et aI, 1995, Plant Mol. BioI. 29: 995-1004).

The term "constitutive" as used herein does not necessarily indicate that a gene under control of the constitutive regulatory region is expressed at the same level in all cell types, but that the gene is expressed in a wide range of cell types even though variation in abundance is often observed. Constitutive regulatory elements may be coupled with other sequences to further enhance the transcription and/or translation of the nucleotide sequence to which they are operatively linked. For example, the CPMV-HT system is derived from the untranslated regions of the Cowpea mosaic virus (CPMV) and demonstrates enhanced translation of the associated coding sequence. By "native" it is meant that the nucleic acid or amino acid sequence is naturally occurring, or "wild type". By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.

The chimeric protein or polypeptide may be expressed in an expression system comprising a viral based, DNA or RNA, expression system, for example but not limited to, a comovirus-based expression cassette and geminivirus-based amplification element.

The expression system as described herein may comprise an expression cassette based on a bipartite virus, or a virus with a bipartite genome. For example, the bipartite viruses may be of the Comoviridae family. Genera of the Comoviridae family include Comovirus, Nepovirus, Fabavirus, Cheravirus and Sadwavirus. Comoviruses include Cowpea mosaic virus (CPMV), Cowpea severe mosaic virus (CPSMV), Squash mosaic virus (SqMV), Red clover mottle virus (RCMV), Bean pod mottle virus (BPMV), Turnip ringspot virus (TuRSV), Broad bean true mosaic virus (BBtMV), Broad bean stain virus (BBSV), Radish mosaic virus (RaMV). Examples of comoviruse RNA-2 sequences comprising enhancer elements that may be useful for various aspects of the invention include, but are not limited to: CPMV RNA-2 (GenBank Accession No. NC_003550), RCMV RNA-2 (GenBank Accession No. NC_003738), BPMV RNA-2 (GenBank Accession No. NC_003495), CPSMV RNA-2 (GenBank Accession No.NC_003544), SqMV RNA-2 (GenBank Accession No.NC_003800), TuRSV RNA-2 (GenBank Accession No. NC_013219.1). BBtMV RNA-2 (GenBank Accession No. GU810904), BBSV RNA2 (GenBank Accession No. FJ028650), RaMV (GenBank Accession No. NC_003800)

Segments of the bipartite comoviral RNA genome are referred to as RNA-1 and RNA-2. RNA-1 encodes the proteins involved in replication while RNA-2 encodes the proteins necessary for cell-to-cell movement and the two capsid proteins. Any suitable comovirus-based cassette may be used including CPMV, CPSMV, SqMV, RCMV, or BPMV, for example, the expression cassette may be based on CPMV.

"Expression cassette" refers to a nucleotide sequence comprising a nucleic acid of interest under the control of, and operably (or operatively) linked to, an appropriate promoter or other regulatory elements for transcription of the nucleic acid of interest in a host cell.

The expression systems may also comprise amplification elements from a geminivirus for example, an amplification element from the bean yellow dwarf virus (BeYDV). BeYDV belongs to the Mastreviruses genus adapted to dicotyledonous plants. BeYDV is monopartite having a single-strand circular DNA genome and can replicate to very high copy numbers by a rolling circle mechanism. BeYDV-derived DNA replicon vector systems have been used for rapid high-yield protein production in plants.

As used herein, the phrase "amplification elements" refers to a nucleic acid segment comprising at least a portion of one ore more long intergenic regions (LIR) of a geminivirus genome. As used herein, "long intergenic region" refers to a region of a long intergenic region that contains a rep binding site capable of mediating excision and replication by a geminivirus Rep protein. In some aspects, the nucleic acid segment comprising one or more LIRs, may further comprises a short intergenic region (SIR) of a geminivirus genome. As used herein, "short intergenic region" refers to the complementary strand (the short IR (SIR) of a Mastreviruses). Any suitable geminivirus-derived amplification element may be used herein. See, for example, WO2000/20557; WO2010/025285; Zhang X. et al. (2005, Biotechnology and Bioengineering, Vol. 93, 271-279), Huang Z. et al. (2009, Biotechnology and Bioengineering, Vol. 103, 706-714), Huang Z. et al.(2009, Biotechnology and Bioengineering, Vol. 106, 9-17);

The chimeric protein or chimeric polypeptide may be produced as a transcript from a chimeric nucleotide sequence, and the chimeric protein or chimeric polypeptide cleaved following synthesis, and as required, associated to form a multimeric protein. Therefore, a chimeric protein or a chimeric polypeptide also includes a protein or polypeptide comprising subunits that are associated via disulphide bridges (i.e. a multimeric protein). For example, a chimeric polypeptide comprising amino acid sequences from two or more than two sources may be processed into subunits, and the subunits associated via disulphide bridges to produce a chimeric protein or chimeric polypeptide.

The chimeric virus protein according to various embodiments of the present invention comprises a transmembrane domain and ctyoplasmic tail (TM/CT) from an influenza HA. The TM/CT can be the native HA TM/CT from any type, subtype of influenza virus, including, for example, B, H1, H2, H3, H4, H5, H6, H7, H8, H9, H10, H11, H12, H13, H14, H15 and H16 types or subtypes. Non limiting examples of HI, H3, H5 or B types or subtypes include the A/NewCaledonia/20/99 subtype (H1N1), the H1 A/California/04/09 subtype (H1N1), the A/Indonesia/5/05 sub-type (H5N1), the A/Brisbane/59/2007, the B/Florida/4/2006, and the H3 A/Brisbane/10/2007 (see for example WO2009/009876; WO 2009/076778; WO 2010/003225; WO 2010/003235). Further, the TM/CT can be of any of those HA TM/CT in which 1, 2, 3, 4 or 5 amino acids have been deleted, or upon which any kind of spacer or linker sequence of 1, 2, 3, 4 or 5 amino acids have been added.

Preferably, the TM/CT is from H5 or H3, for example but not limited to A/Indonesia/5/05 sub-type (H5N1; "H5/Indo"; GenBank Accession No. ABW06108.1), H5 A/Vietnam/1194/2004(A-Vietnam; GenBank Accession No. ACR48874.1), H5 A/Anhui/1/2005 (A-Anhui; GenBank Accession No. ABD28180.1); H3 A/Brisbane/10/2007 ("H3/Bri"; GenBank Accession No. ACI26318.1), H3 A/Wisconsin/67/2005(A-WCN; GenBank Accession No. ABO37599.1). The TM/CT of boundaries of several H3 and H5 sequences are described in WO 2010/148511. Non-limiting examples of amino acid sequences for the TM/CT include SEQ ID NO:41 and 42, and any nucleotide sequence that encodes the amino acid sequence of SEQ ID NO:41 or 42

Amino acid variation is tolerated in hemagglutinin of influenza viruses. Such variation provides for new strains that are continually being identified. Infectivity between the new strains may vary. However, formation of hemagglutinin trimers, which subsequently form VLPs is maintained. The present invention, therefore, provides for a chimeric virus protein comprising a hemagglutinin amino acid sequence, or a nucleic acid encoding a chimeric virus protein comprising a hemagglutinin amino acid sequence, that forms VLPs in a plant, and includes known sequences and variant HA sequences that may develop.

The term "virus like particle" (VLP), or "virus-like particles" or "VLPs" refers to structures that self-assemble and comprise structural proteins such as chimeric virus protein. VLPs and chimeric VLPs are generally morphologically and antigenically similar to virions produced in an infection, but lack genetic information sufficient to replicate and thus are non-infectious. VLPs and chimeric VLPs may be produced in suitable host cells including plant host cells. Following extraction from the host cell and upon isolation and further purification under suitable conditions, VLPs and chimeric VLPs may be purified as intact structures.

The chimeric VLPs of the present invention may be produced in a host cell that is characterized by lacking the ability to sialylate proteins, for example a plant cell, an insect cell, fungi, and other organisms including sponge, coelenterara, annelida, arthoropoda, mollusca, nemathelminthea, trochelmintes, plathelminthes, chaetognatha, tentaculate, chlamydia, spirochetes, gram-positive bacteria, cyanobacteria, archaebacteria, or the like. See, for example Gupta et al., 1999. Nucleic Acids Research 27:370-372; Toukach et al., 2007. Nucleic Acids Research 35:D280-D286; Nakahara et al., 2008. Nucleic Acids Research 36:D368-D371.

The invention also provides chimeric VLPs that obtain a lipid envelope from the plasma membrane of the cell in which the chimeric VLPs are expressed. For example, if the chimeric virus is expressed in a plant-based system, the resulting chimeric VLP may obtain a lipid envelope from the plasma membrane of the plant cell.

Generally, the term "lipid" refers to a fat-soluble (lipophilic), naturally-occurring molecule. A chimeric VLP produced in a plant according to some aspects of the invention may be complexed with plant-derived lipids. The plant-derived lipids may be in the form of a lipid bilayer, and may further comprise an envelope surrounding the VLP. The plant-derived lipids may comprise lipid components of the plasma membrane of the plant where the VLP is produced, including phospholipids, tri-, di- and monoglycerides, as well as fat-soluble sterol or metabolites comprising sterols. Examples include phosphatidylcholine (PC), phosphatidylethanolamine (PE), phosphatidylinositol, phosphatidylserine, glycosphingolipids, phytosterols or a combination thereof. A plant-derived lipid may alternately be referred to as a 'plant lipid'. Examples ofphytosterols include campesterol, stigmasterol, ergosterol, brassicasterol, delta-7-stigmasterol, delta-7-avenasterol, daunosterol, sitosterol, 24-methylcholesterol, cholesterol or beta-sitosterol - see, for example, Mongrand et ai., 2004. As one of skill in the art would understand, the lipid composition of the plasma membrane of a cell may vary with the culture or growth conditions of the cell or organism, or species, from which the cell is obtained. Generally, beta-sitosterol is the most abundant phytosterol.

Cell membranes generally comprise lipid bilayers, as well as proteins for various functions. Localized concentrations of particular lipids may be found in the lipid bilayer, referred to as 'lipid rafts'. These lipid raft microdomains may be enriched in sphingolipids and sterols. Without wishing to be bound by theory, lipid rafts may have significant roles in endo and exocytosis, entry or egress of viruses or other infectious agents, inter-cell signal transduction, interaction with other structural components of the cell or organism, such as intracellular and extracellular matrices.

The VLP produced within a plant may induce an chimeric virus proteins comprising plant-specific N-glycans. Therefore, this invention also provides for a VLP comprising chimeric virus proteins having plant specific N-glycans.

Furthermore, modification of N-glycan in plants is known (see for example U.S. 60/944,344; and chimeric virus proteins having modified N-glycans may be produced. Chimeric virus proteins comprising a modified glycosylation pattern, for example with reduced fucosylated, xylosylated, or both, fucosylated and xylosylated, N-glycans may be obtained, or chimeric virus proteins having a modified glycosylation pattern may be obtained, wherein the protein lacks fucosylation, xylosylation, or both, and comprises increased galatosylation. Furthermore, modulation of post-translational modifications, for example, the addition of terminal galactose may result in a reduction of fucosylation and xylosylation of the expressed chimeric virus proteins when compared to a wild-type plant expressing chimeric virus proteins.

For example, which is not to be considered limiting, the synthesis of chimeric virus proteins having a modified glycosylation pattern may be achieved by co-expressing the chimeric virus protein along with a nucleotide sequence encoding beta-1.4galactosyltransferase (GalT), for example, but not limited to mammalian GalT, or human GalT however GalT from another sources may also be used. The catalytic domain of GalT may also be fused to a CTS domain (i.e. the cytoplasmic tail, transmembrane domain, stem region) of N-acetylglucosaminyl transferase (GNT1), to produce a GNT1-GalT hybrid enzyme, and the hybrid enzyme may be co-expressed with chimeric virus protein. The chimeric virus protein may also be co-expressed along with a nucleotide sequence encoding N-acetylglucosaminyltrasnferase III (GnT-III), for example but not limited to mammalian GnT-III or human GnT-III, GnT-III from other sources may also be used. Additionally, a GNT1-GnT-III hybrid enzyme, comprising the CTS of GNT1 fused to GnT-III may also be used.

Therefore the present invention also provides VLP's comprising chimeric virus protein having modified N-glycans.

Without wishing to be bound by theory, the presence of plant N-glycans on chimeric virus protein may stimulate the immune response by promoting the binding of chimeric virus protein by antigen presenting cells. Stimulation of the immune response using plant N glycan has been proposed by Saint-Jore-Dupas et al. (2007). Furthermore, the conformation of the VLP may be advantageous for the presentation of the antigen, and enhance the adjuvant effect of VLP when complexed with a plant derived lipid layer.

VLPs may be assessed for structure and size by, for example, hemagglutination assay, electron microscopy, or by size exclusion chromatography.

For size exclusion chromatography, total soluble proteins may be extracted from plant tissue by homogenizing (Polytron) sample of frozen-crushed plant material in extraction buffer, and insoluble material removed by centrifugation. Precipitation with ice cold acetone or PEG may also be of benefit. The soluble protein is quantified, and the extract passed through a Sephacryl™ column, for example a Sephacryl™ S500 column. Blue Dextran 2000 may be used as a calibration standard. Following chromatography, fractions may be further analyzed by immunoblot to determine the protein complement of the fraction.

The separated fraction may be for example a supernatant (if centrifuged, sedimented, or precipitated), or a filtrate (if filtered), and is enriched for proteins, or suprastructure proteins, such as for example rosette-like structures or higher-order, higher molecular weight, particles such as VLPs. The separated fraction may be further processed to isolate, purify, concentrate or a combination thereof, the proteins, or suprastructure proteins, by, for example, additional centrifugation steps, precipitation, chromatographic steps (e.g. size exclusion, ion exchange, affinity chromatography), tangential flow filtration, or a combination thereof. The presence of purified proteins, or suprastructure proteins, may be confirmed by, for example, native or SDS-PAGE, Western analysis using an appropriate detection antibody, capillary electrophoresis, electron microscopy, or any other method as would be evident to one of skill in the art.

Elution profiles may vary depending on the elution conditions used. Figures 7, 8O and 11B show an example of an elution profile of a size exclusion chromatography analysis of a plant extract comprising chimeric VLPs. In this case, VLPs comprising chimeric HIV, chimeric rabies G and chimeric VZV virus trimeric surface proteins elute in the void volume of the column, rosettes and high molecular weight structures elute from about fractions 13 to 14, and lower molecular weight, or soluble form of the chimeric virus trimeric surface proteins elute in fractions from about 15 to about 17.

The VLPs may be purified or extracted using any suitable method for example chemical or biochemical extraction. VLPs are relatively sensitive to desiccation, heat, pH, surfactants and detergents. Therefore it may be useful to use methods that maximize yields, minimize contamination of the VLP fraction with cellular proteins, maintain the integrity of the proteins, or VLPs, and, where required, the associated lipid envelope or membrane, methods of loosening the cell wall to release the proteins, or VLP. For example, methods that produce protoplast and/or spheroplasts may be used (see for example WO 2011/035422, to obtain VLPs as described herein. Minimizing or eliminating the use of detergence or surfactants such for example SDS or Triton X-100 may be beneficial for improving the yield of VLP extraction. VLPs may be then assessed for structure and size by, for example, electron microscopy, or by size exclusion chromatography as mentioned above.

The one or more than one or more chimeric genetic constructs of the present invention may be expressed in any suitable plant host that is transformed by the nucleotide sequence, or constructs, or vectors of the present invention. Examples of suitable hosts include, but are not limited to, agricultural crops including alfalfa, canola, *Brassica* spp., maize, *Nicotiana* spp., alfalfa, potato, ginseng, pea, oat, rice, soybean, wheat, barley, sunflower, cotton and the like.

The one or more chimeric genetic constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. Non-limiting examples of suitable 3' regions are the 3' transcribed nontranslated regions containing a polyadenylation signal of *Agrobacterium* tumor inducing (Ti) plasmid genes, such as the nopaline synthase (NOS) gene, plant genes such as the soybean storage protein genes, the small subunit of the ribulose-I, 5-bisphosphate carboxylase gene (ssRUBISCO; US 4,962,028; the promoter used in regulating plastocyanin expression, described in US 7,125,978).

One or more of the chimeric genetic constructs of the present invention may also include further enhancers, either translation or transcription enhancers, as may be required. Enhancers may be located 5' or 3' to the sequence being transcribed. Enhancer regions are well known to persons skilled in the art, and may include an ATG initiation codon, adjacent sequences or the like. The initiation codon, if present, may be in phase with the reading frame ("in frame") of the coding sequence to provide for correct translation of the transcribed sequence.

The constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff (J Virol Meth, 105:343-348, 2002,), U.S. Pat. Nos. 4,945,050; 5,036,006; and 5,100,792, U.S. patent application Ser. Nos. 08/438,666, filed May 10, 1995, and 07/951,715, filed Sep. 25, 1992, ).

As described below, transient expression methods may be used to express the constructs of the present invention (see Liu and Lomonossoff, 2002, Journal of Virological Methods, 105:343-348). Alternatively, a vacuum-based transient expression method, as described by Kapila et al., 1997, may be used. These methods may include, for example, but are not limited to, a method of Agro-inoculation or Agro-infiltration, syringe infiltration, however, other transient methods may also be used as noted above. With Agro-inoculation, Agro-infiltration, or synringe infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacteria* infect and transfer t-DNA copies into the cells. The t-DNA is episomally transcribed and the mRNA translated, leading to the production of the protein of interest in infected cells, however, the passage of t-DNA inside the nucleus is transient.

To aid in identification of transformed plant cells, the constructs of this invention may be further manipulated to include plant selectable markers. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

Also considered part of this invention are transgenic plants, plant cells or seeds containing the chimeric gene construct of the present invention. Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue cultures.

The present invention discloses nucleotide sequences:

**Table 1. List of Sequence Identification numbers.**

| **SEQ ID NO:** | **Description** | **Table/Figure** |
|---|---|---|
| 1 | Consensus nucleic acid sequence of HIV ConS ΔCFI (Native signal peptide in bold, native transmembrane and cytosolic domains are underlined) | Figure 1 A |
| 2 | IF-ApaI-SpPDI.c | Figure 1B |
| 3 | SpPDI-HIV gp145.r | Figure 1C |
| 4 | IF-SpPDI-gp145.c | Figure 1D |
| 5 | WtdTm-gp145.r | Figure 1E |
| 6 | Expression cassette number 995, from PacI (upstream of the promoter) to AscI (immediately downstream of the NOS terminator). Eliminated SbfI restriction site is bolded. PDISP-HIV ConS ΔCFI is underlined. | Figure 1F |
| 7 | Amino acid sequence of PDISP-HIV ConS ΔCFI | Figure 1G |
| 8 | IF-H3dTm+gp145.r | Figure 2A |
| 9 | Gp145+H3dTm.c | Figure 2B |
| 10 | H3dTm.r | Figure 2C |
| 11 | Expression cassette number 997, from PacI (upstream of the promoter) to AscI (immediately downstream of the NOS terminator). Eliminated SbfI restriction site is bolded. PDISP-HIV Con S ΔCFI-A/Brisbane/10/2007 H3 TM+CT is underlined. | Figure 2D |
| 12 | Amino acid sequence of PDISP-HIV ConS ΔCFI-A/Brisbane/10/2007 H3 TM+CT | Figure 2E |
| 13 | IF-H5dTm+gp145.r | Figure 3A |
| 14 | Gp145+H5dTm.c | Figure 3B |
| 15 | IF-H5dTm.r | Figure 3C |
| 16 | Expression cassette number 999, from PacI (upstream of the promoter) to AscI (immediately downstream of the NOS terminator). Eliminated SbfI restriction site is bolded. PDISP-HIV ConS ΔCFI-A/Indonesia/5/2005 H5 TM+CTis underlined. | Figure 3D |
| 17 | Amino acid sequence of PDISP-HIV ConS ΔCFI-A/Indonesia/5/2005 H5 TM+CT | Figure 3E |
| 18 | Expression cassette number 172, from XmaI (upstream of the plastocyanin promoter) to EcoRI (immediately downstream of the plastocyanin terminator). TBSV P19 nucleic acid sequence is underlined. | Figure 4A |
| 19 | Amino acid sequence of TBSV P19 suppressor of silencing | Figure 4B |
| 20 | IF-wtSp-VZVgE.c | Figure 10A |
| 21 | IF-H5dTm+VZVgE.r | Figure 10B |
| 22 | Synthesized VZV gE gene (corresponding to nt 3477-5348 from Genebank accession number AY013752.1) | Figure 10C |
| 23 | VZVgE+H5dTm.c | Figure 10D |
| 24 | Expression cassette number 946, from PacI (upstream of the promoter) to AscI (immediately downstream of the NOS terminator).VZV gE-A/Indonesia/5/2005 H5 TM+CT underlined | Figure 10E |
| 25 | Amino acid sequence of VZV gE- A/Indonesia/5/2005 H5 TM+CT | Figure 10F |
| 26 | IF-wtSp-SARSgS.c | Figure 12B |
| 27 | IF-H5dTm+SARSgS.r | Figure 12C |
| 28 | synthesized SARS gS gene (corresponding to nt 21492-25259 from Genebank accession number AY278741.1) (Native signal peptide in bold, native transmembrane and cytosolic domains are underlined) | Figure 12D |
| 29 | SARSgS+H5dTm.c | Figure 12E |
| 30 | Expression cassette number 916, from PacI (upstream of the promoter) to AscI (immediately downstream of the NOS terminator).SARS gS-A/Indonesia/5/2005 H5 TM+CT underlined | Figure 12F |
| 31 | Amino acid sequence of SARS gS- A/Indonesia/5/2005 H5 TM+CT | Figure 12G |
| 32 | IF-RabG-S2+4.c | Figure 8B |
| 33 | RabG+H5dTm.r | Figure 8C |
| 34 | Synthesized Rab G gene (corresponding to nt 3317-4891 from Genebank accession number EF206707) (Native signal peptide in bold, native transmembrane and cytosolic domains are underlined) | Figure 8D |
| 35 | IF-H5dTm.c | Figure 8E |
| 36 | Construct 141 from left to right t-DNA (underlined).2X35S-CPMV-HT-PDISP-NOS expression system with Plastocyanine-P19-Plastocyanine silencing inhibitor expression cassette | Figure 8F |
| 37 | Expression cassette number 1074 from 2X35S promoter to NOS terminator.PDISP-Rab G -A/Indonesia/5/2005 H5 TM+CT is underlined. | Figure 8H |
| 38 | Amino acid sequence ofPDISP-Rab G- A/Indonesia/5/2005 H5 TM+CT | Figure 8I |
| 39 | Construct 144 from left to right t-DNA (underlined).2X35S-CPMV-HT-PDISP-NOS into BeYDV + replicaseexpression system with Plastocyanine-P19-Plastocyanine silencing inhibitor expression cassette | Figure 8J |
| 40 | Expression cassette number 1094 from right to left BeYDV LIR.PDISP-Rab G -A/Indonesia/5/2005 H5 TM+CT is underlined. | Figure 8L |
| 41 | H5 (A/Indonesia/05/2005) TM/CT: QILSIYSTVASSLALAIMMAGLSLWMCSNGSLQCRICI | |
| 42 | H3 (A/Brisbane/10/2007) TM/CT: DWILWISFAISCFLLCVALLGFIMWACQKGNIRCNICI | |
| 43 | IF-Opt_EboGP.s2+4c | Figure 13A |
| 44 | H5iTMCT+Opt_EboGP.r | Figure 13B |
| 45 | Optimized synthesized GPgene | Figure 13C |
| 46 | Opt_EboGP+H5iTMCT.c | Figure 13D |
| 47 | Construct 1192 | Figure 13F |
| 48 | Expression cassette number 1366 | Figure 13G |
| 49 | Amino acid sequence of PDISP-GP from Zaire 95 Ebolavirus-H5 TM+CT from A/Indonesia/5/2005 | Figure 13 H |

The present invention will be further illustrated in the following examples.

### Examples

### Constructs

**Table 2: Constructs comprising sequences encoding HIV protein**

| Expression System | Amplification System | Sp | 5' | Protein | 3' | Nptll (Ori tDNA) | Plasto-P19 (Ori tDNA) | No. Const |
|---|---|---|---|---|---|---|---|---|
| CPMV HT | - | WtSp | - | Gp145ΔCFI | wt TM+CT | L→R | - | 994 |
| CPMV HT | - | Sp PDI | - | Gp145ΔCFI | wt TM+CT | L→R | - | 995 |
| CPMV HT | - | WtSp | - | Gp145ΔCFI | H3 A/Bri TM+CT | L→R | - | 996 |
| CPMV HT | - | Sp PDI | - | Gp145ΔCFI | H3 A/Bri TM+CT | L→R | - | 997 |
| CPMV HT | - | WtSp | - | Gp145ΔCFI | H5 A/Indo TM+CT | L→R | - | 998 |
| CPMV HT | - | Sp PDI | - | Gp145ΔCFI | H5 A/Indo TM+CT | L→R | - | 999 |
| CPMV HT | BeYDV+rep | Sp PDI | - | Gp145ΔCFI | wt TM+CT | - | L→R | 985 |
| CPMV HT | BeYDV+rep | Sp PDI | - | Gp145ΔCFI | H3 A/Bri TM+CT | - | L→R | 987 |
| CPMV HT | BeYDV+rep | Sp PDI | - | Gp145ΔCFI | H5 A/Indo TM+CT | - | L→R | 989 |

**Table 3: Constructs comprising sequences encoding rabies virus protein**

| Expression System | Amplification System | Sp | 5' | Protein | 3' | Nptll (Or1 tDNA) | Plasto-P19 (Ori tDNA) | No. Const |
|---|---|---|---|---|---|---|---|---|
| CPMV HT | - | wt SP | - | Rabies G | - | - | L→R | 1070 |
| CPMV HT | - | Sp PDI | - | Rabies G | - | - | L→R | 1071 |
| CPMV HT | BeYDV+rep | wt SP | - | Rabies G | - | - | L→R | 1090 |
| CPMV HT | BeYDV+rep | Sp PDI | - | Rabies G | - | - | L→R | 1091 |
| CPMV HT | - | Sp PDI | - | Rabies G | H5 A/lndo TM+CT | - | L→R | 1074 |
| CPMV HT | BeYDV+rep | Sp PDI | - | Rabies G | H5 A/lndo TM+CT | - | L→R | 1094 |
| CPMV HT | BeYDV+rep | Sp PDI | - | Rabies G (A447S) | - | - | L→R | 1072 |
| CPMV HT | BeYDV+rep | Sp PDI | - | Rabies G (A447S) | - | - | L→R | 1092 |
| CPMV HT | - | Sp PDI | - | Rabies G (M44I+V392G) | - | - | L→R | 1073 |
| CPMV HT | BeYDV+rep | Sp PDI | - | Rabies G (M44I+V392G) | - | - | L→R | 1093 |
| CPMVHT | | Sp PDI | - | Rabies G (M44I+V392G) | H5 A/Indo TM+CT | - | L→R | 1075 |
| CPMVHT | BeYDV+rep | Sp PDI | - | Rabies G (M44I+V392G) | H5 A/Indo TM+CT | - | L→R | 1095 |

**Table 4: Construct comprising sequences encoding VZV protein**

| Expression System | Amplification System | Sp | 5' | Protein | 3' | Nptll (Ori Tdna) | Plasto-P19 (Ori tDNA) | No. Const |
|---|---|---|---|---|---|---|---|---|
| CPMV HT | - | WtSp | - | VZV gE | wt TM+CT | L→R | | 944 |
| CPMV HT | - | Sp PDI | - | VZV gE | wt TM+CT | L→R | - | 945 |
| CPMV HT | - | WtSp | - | VZV gE | HS A/Indo TM+CT | L→R | - | 946 |
| CPMV HT | - | Sp PDI | - | VZV gE | HS A/Indo TM+CT | L→R | - | 947 |

**Table 5: Construct comprising sequences encoding SARS protein**

| Expression System | Amplification System | Sp | 5' | Protein | 3' | Nptll (Ori tDNA) | Plasto-P19 (OritDNA) | No. Const |
|---|---|---|---|---|---|---|---|---|
| CPMV HT | | WtSp | - | SARS gS | wt TM+CT | L→R | | 914 |
| CPMV HT | - | Sp PDI | - | SARS gS | wt TM+CT | L→R | - | 915 |
| CPMV HT | | WtSp | - | SARS gS | H5 A/Indo TM+CT | L→R | - | 916 |
| CPMV HT | | Sp PDI | - | SARS gS | H5 A/Indo TM+CT | L→R | - | 917 |

### Example 1: Assembly of expression cassettes with HIV protein

### 2X35S-CPMV HT-PDISP-HIV ConS ΔCFI-NOS (construct number 995)

A sequence encoding alfalfa PDI signal peptide fused to HIV ConS ΔCFI with native transmembrane domain and cytosolic tail was cloned into 2X35S-CPMV-HT expression system as follows. First, the nucleic acid sequence of the HIV ConS ΔCFI gene, comprising the native signal peptide and transmembrane and cytoplasmic domains was synthesized by GeneArt AG (Regensburg, Germany) according to the sequences disclosed in Liao et al, (2006, Virology 353: 268-282). The nucleic acid sequence of HIV ConS ΔCFI is presented in Figure 1A (SEQ ID NO: 1). The signal peptide of alfalfa protein disulfide isomerase (PDISP) (nucleotides 32-103; Accession No. Z11499) was linked to the HIV ConS ΔCFI by the PCR-based ligation method presented by Darveau et al. (Methods in Neuroscience 26: 77-85 (1995)). In a first round of PCR, a fragment containing PDISP was amplified using primers IF-ApaI-SpPDLc (Figure 1B, SEQ ID NO: 2) and SpPDI-HIV gp145.r (Figure 1C, SEQ ID NO: 3) with construct number 540 (see Figure 52, SEQ ID NO: 61 of WO 2010/003225. which is incorporated herein by reference, for the sequence of construct number 540) as template. A second fragment containing ConS ΔCFI without the native signal peptide was amplified using primers IF-SpPDI-gp145.c (Figure 1D, SEQ ID NO: 4) and WtdTm-gp145.r (Figure 1E, SEQ ID NO: 5) using the synthesized ConS ΔCFI segment (Figure 1A, SEQ ID NO: 1) as template. In a second round of PCR, primers IF-ApaI-SpPDI.c (Figure 1B, SEQ ID NO: 2) and WtdTm-gp145.r (Figure 1E, SEQ ID NO: 5) were used along with both PCR product from the first round of PCR as template. The resulting PCR product was digested with ApaI restriction enzyme and cloned into a modified 972 construct digested with ApaI-StuI. The modified 972 acceptor plasmid (see Figure 94, SEQ ID NO: 134 of WO 2010/003225, for original sequence of construct number 972) was treated to eliminate a SbfI restriction site upstream of the 2X35S promoter. The SbfI site was eliminated by digesting plasmid 972 with SbfI, treating the resulting plasmid with T4 DNA polymerase to remove the 3' overhang and religating the treated plasmid, resulting in the modified 972 plasmid without SbfI site. The resulting construct was given number 995 (Figure 1F, SEQ ID NO: 6). The amino acid sequence of PDISP-HIV ConS ΔCFI is presented in Figure 1G (SEQ ID NO: 7). The 995 plasmid representation is presented in Figure 1H. This construct does not encode an M1 protein.

### 2X35S-CPMV HT -PDISP-HIV ConS ΔCFI+ H3 A/Brisbane/10/2007 (TmD + Cyto tail)-NOS (construct number 997)

A sequence encoding alfalfa PDI signal peptide fused to HIV ConS ΔCFI and to the transmembrane and cytosolic domains of H3 A/Brisbane/10/2007 was cloned into 2X35S-CPMV-HT expression system using the PCR-based ligation method presented by Darveau et al. (Methods in Neuroscience 26: 77-85 (1995)). In a first round of PCR, a fragment comprising PDISP-HIV ConS ΔCFI without the native transmembrane and cytoplasmic domains was amplified using primers IF-ApaI-SpPDI.c (Figure 1B, SEQ ID NO: 2) and IF-H3dTm+gp145.r (Figure 2A, SEQ ID NO: 8), using construct number 995 (Figure 1F, SEQ ID NO: 6) as template. A second fragment containing the transmembrane and cytosolic domains of H3 A/Brisbane/10/2007 was amplified using primers Gp145+H3dTm.c (Figure 2B, SEQ ID NO: 9) and H3dTm.r (Figure 2C, SEQ ID NO: 10), using construct number 776 (see Figure 60, SEQ ID NO: 69 of WO 2010/003225, for construct number 776 sequence) as template. The PCR products from both amplifications were then mixed and used as template for a second round of amplification using IF-ApaI-SpPDI.c (Figure 1B, SEQ ID NO: 2) and H3dTm.r (Figure 2C, SEQ ID NO: 10) as primers. The product of the second PCR was then digested with ApaI and cloned into construct number 995 (Figure 1F, SEQ ID NO: 6) digested with ApaI and StuI restriction enzymes. The resulting construct was given number 997 (Figure 2D, SEQ ID NO: 11). The amino acid sequence of PDISP-HIV ConS ΔCFI-A/Brisbane/10/2007 H3 TM+CT is presented in Figure 2E (SEQ ID NO: 12). The 997 plasmid representation is presented in Figure 2F. This construct does not encode an M1 protein.

### 2X35S-CPMV HT-PDISP-HIV ConS ΔCFI-H5 A/Indonesia/5/2005 (TmD + Cyto tail)-NOS (construct number 999)

A sequence encoding alfalfa PDI signal peptide fused to HIV ConS ΔCFI and to the transmembrane and cytosolic domains of H5 A/Indonesia/5/2005 was cloned into 2X35S-CPMV-HT expression system as follows using the PCR-based ligation method presented by Darveau et al. (Methods in Neuroscience 26: 77-85 (1995)). In a first round of PCR, a fragment containing PDISP-HIV ConS ΔCFI without the native transmembrane and cytoplasmic domains was amplified using primers IF-ApaI-SpPDI.c (Figure 1B, SEQ ID NO: 2) and IF-H5dTm+gp145.r (Figure 3A, SEQ ID NO: 13), using construct number 995 (Figure 1F, SEQ ID NO: 6) as template. A second fragment containing the transmembrane and cytoplasmic domains of H5 A/Indonesia/5/2005 was amplified using primers Gp145+H5dTm.c (Figure 3B, SEQ ID NO: 15) and IF-H5dTm.r (Figure 3C, SEQ ID NO: 15), using construct number 972 (see Figure 94, SEQ ID NO: 134 of WO 2010/003225 for the sequence of construct number 972) as template. The PCR products from both amplifications were then mixed and used as template for a second round of amplification using IF-ApaI-SpPDI.c (Figure 1B, SEQ ID NO: 2) and IF-H5dTm.r (Figure 3C, SEQ ID NO: 15) as primers. The product of the second PCR was then digested with ApaI and cloned into construct number 995 (Figure 1F, SEQ ID NO: 6) digested with with ApaI and StuI restriction enzymes. The resulting construct was given number 999 (Figure 3D, SEQ ID NO: 16). The amino acid sequence of PDISP-HIV ConS ΔCFI- A/Indonesia/5/2005 H5 TM+CT. TM+CT is presented in Figure 3E (SEQ ID NO: 17). The 999 plasmid representation is presented in Figure 3F. This construct does not encode an M1 protein.

### Plastocyanin-P19-plastocyanin (construct number 172)

A sequence encoding P19 suppressor of silencing from Tomato Bushy Stunt Virus (TBSV) was cloned between the alfalfa plastocyanin promoter and 3'UTR and terminator as follows. Construct number R472 (see WO 2010/003225 A1, for assembly and Figure 86 of WO 2010/003225 A1 for a representation of R472 plasmid) was digested with restriction enzymes DraIII (84 base pairs upstream of initial ATG) and SacI (9 base pairs downstream of the stop codon) to remove a fragment comprising 84 pb from the alfalfa plastocyanin promoter and the sequence coding for TBSV P19 suppressor of silencing. The resulting fragment was cloned into construct 540 (see WO 2010/003225, for assembly and figure 6 of the same patent for a representation of 540 plasmid) previously digested with DraIII and SacI. The resulting construct was given number 172 (SEQ ID NO: 4A, Figure 18). The amino acids sequence of TBSV P19 protein is presented in Figure 4B (SEQ ID NO: 19). A 172 plasmid representation is presented in Figure 4C.

### Preparation of plant biomass, inoculum and agroinfiltration

The terms "biomass" and "plant matter" as used herein are meant to reflect any material derived from a plant. Biomass or plant matter may comprise an entire plant, tissue, cells, or any fraction thereof. Further, biomass or plant matter may comprise intracellular plant components, extracellular plant components, liquid or solid extracts of plants, or a combination thereof. Further, biomass or plant matter may comprise plants, plant cells, tissue, a liquid extract, or a combination thereof, from plant leaves, stems, fruit, roots or a combination thereof. A portion of a plant may comprise plant matter or biomass.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest. Unless otherwise specified, all infiltrations were performed as co-infiltration with strain AGL1/172 in a 1:1 ratio.

*A. tumefaciens* strains comprising the various constructs as described herein are referred to by using an "AGL1"prefix. For example A. *tumefaciens* comprising construct number 995 (see Figure 1H, is termed "AGL1/995".

### Leaf harvest and total protein extraction

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

### Protein analysis and immunoblotting

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with a goat polyclonal anti-gp120 primary antibody (Abcam, ab21179) diluted 1:2500 in 2 µg/ml in 2% skim milk in TBS-Tween 20 0.1%. Chemiluminescence detection was carried on after incubation with peroxidase-conjugated donkey anti-goat secondary antibodies (JIR 705-035-147), diluted 1:10000 in 2% skim milk in TBS-Tween 20 0.1%. Immunoreactive complexes were detected by chemiluminescence using luminol as the substrate (Roche Diagnostics Corporation)

### Example 2: Expression of native and chimeric HIV envelope proteins in plants

HIV ConS ΔCFI is a consensus HIV group M envelope protein with shorten variable loops, deletion of the cleavage site, the fusion domain and an immunodominant region in gp41. It was demonstrated to elicit cross-subtype neutralizing antibodies of similar or greater breadth and titer than the wild-type envelope proteins in guinea pigs (Liao et al., Virology (2006) 353: 268-282).

Figure 5 presents the constructs used in this study. In construct number 995, the coding region for the mature HIV ConS ΔCFI (later referred to as Env), comprising the native TM/CT, was placed under the control of the CPMV-HT expression system. In constructs number 997 and 999, the TM/CT domains of HIV Env were replaced by those of influenza hemagglutinin (HA) from A/Brisbane/10/2007 (H3N2) and A/Indonesia/05/2005 (H5N1), respectively. In all cases, a signal peptide of plant origin - from the alfalfa protein disulfide isomerase (PDI) protein - replaced the native HIV Env protein signal peptide.

Production of HIV Env from constructs 995, 997 and 999 was compared in agroinfiltrated *Nicotiana benthamiana* plants. Protein extracts from plants infiltrated with AGL1/995, AGL1/997 and AGL1/999 were analyzed by Western blot, and the result are shown in Figure 6, where lanes 1 to 4 are positive controls containing various amounts of recombinant HIV-1 gp160 (ab68171); lane 5, negative control; lanes 6 to 8, proteins extracted from AGL1/995-infiltrated leaves; lanes 9 and 10, proteins extracted from AGL1/997-infiltrated leaves; lanes 11 and 12, proteins extracted from AGL1/999-infiltrated leaves.

As shown in Figure 6, expression of the native HIV Env could not be detected in the conditions used for detection, confirming the very low accumulation level previously reported for HIV Env protein in plants (Rybicki et al., 2010, Plant Biotechnology Journal 8: 620-637). The chimeric forms of Env, in which the TM/CT domains were replaced by that of influenza H3 (construct # 997) or H5 (construct #999) accumulated at much higher levels than the native form. As noted above, The #997 and #999 constructs do not encode an M1 protein.

### Example 3 Chimeric HIV Env assemble into virus-like particles

The capacity of the chimeric HIV Env to assemble into VLP in plant in absence of core or matrix protein was also examined. Protein extracts from AGL1/997 (Env/H3) and AGL1/999 (Env/H5) were subjected to gel filtration chromatography followed by analysis of elution fractions for Env content using goat anti-gp120 antibodies. The Western blot presented in Figure 7A shows that for extracts from AGL1/999-infiltrated plants, chimeric Env/H5 content in elution fractions peaks in fractions 7 to 10, indicating their assembly in very high molecular weight structures of more than 2 million daltons. Examination of the relative protein content in fractions 7 to 18 clearly shows that the great majority of the host proteins eluted in fractions 11 to 18 (Figure 7B). These results show that Env/H5 assembles into high molecular weight structures of size beyond that of the expected molecular weight of the homotrimer. Similar results were obtained for Env/H3 in AGL1/997-infiltrated plants. Taken together these results demonstrate that chimeric HIV Env/H5 accumulated at high level in agroinfiltrated plants and assembled into virus-like particles in absence of core or matrix protein. These results also demonstrate that the fusion of TM/CT domains of influenza HA to non-influenza antigens is sufficient to assemble and release VLPs presenting the non-influenza antigen.

### Example 4: Assembly of expression cassettes with Rabies protein

### C-2X35S-CPMV HT-PDISP-Rabies glycoprotein G (RabG)+H5 A/Indonesia/5/2005 transmembrane domain and cytoplasmic tail (TM+CT)-NOS (Construct number 1074; Figure 8A, 8H).

A sequence encoding Rabies glycoprotein G (RabG)ectodomain fused to the transmembrane and cytosolic domains of H5 A/Indonesia/5/2005 was cloned into 2X35S-CPMV-HT-PDISP-NOS expression system in a plasmid containing Plastocyanine-P19-Plastocyanine expression cassette as follows using the PCR-based ligation method presented by Darveau et al. (Methods in Neuroscience 26: 77-85 (1995)). In a first round of PCR, a fragment containing Rab G ectodomain without the native signal peptide, transmembrane and cytoplasmic domains was amplified using primersIF-RabG-S2+4.c (Figure 8B, SEQ ID NO:32) and RabG+H5dTm.r (Figure 8C, SEQ ID NO:33), using synthesized Rab G gene (corresponding to nt3317-4891 from Genebank accession number EF206707) (Figure 8D, SEQ ID NO:34) as template. A second fragment containing the transmembrane and cytoplasmic domains of H5 A/Indonesia/5/2005 was amplified using primers IF-H5dTm.c (Figure 8E, SEQ ID NO:35) and IF-H5dTm.r (Figure 3C, SEQ ID NO: 15), using construct number 972 (see Figure 94, SEQ ID NO: 134 of WO 2010/003225, for the sequence of construct number 972) as template. The PCR products from both amplifications were then mixed and used as template for a second round of amplification using IF-RabG-S2+4.c (Figure 8B, SEQ ID NO:32)and IF-H5dTm.r (Figure 3C, SEQ ID NO: 15) as primers. The resulting fragment was cloned in-frame with alfalfa PDI signal peptide in 2X35S-CPMV HT-NOS expression system using In-Fusion cloning system by Clontech (Mountain View, CA). Construct 141 (Figure 8F, 8G) was digested with SbfI and StuI restriction enzyme and used for the In-Fusion assembly reaction. Construct number 141 is an acceptor plasmid intended for "In Fusion" cloning of genes of interest in a CPMV HT-based expression cassette. It also incorporates a gene construct for the co-expression of the TBSV P19 suppressor of silencing under the alfalfa Plastocyanin gene promoter and terminator. The vector is a pCAMBIA-based plasmid and the sequence from left to right t-DNA borders is presented in Figure 8F (SEQ ID NO:36). A schematic representation of the vector 141 is presented in Figure 8G.The resulting construct was given number 1074 (Figure 8H, SEQ ID NO: 37). The amino acid sequence of PDISP - Rab G - A/Indonesia/5/2005 H5 TM+CT is presented in Figure 8I (SEQ ID NO:38). The 1074 plasmid representation is presented in Figure 8A.This construct does not encode an M1 protein.

### D-2X35S-PDISP-Rabies glycoprotein G (RabG)+H5 A/Indonesia/5/2005 transmembrane domain and cytoplasmic tail (TM+CT)-NOS intoBeYDV + replicase amplification system (Construct number 1094: Figure 8L, 8M)

A sequence encoding Rabies glycoprotein G (Rab G) ectodomain fused to the transmembrane and cytosolic domains of H5 A/Indonesia/5/2005 was cloned into 2X35S-CPMV-HT-PDISP-NOS into BeYDV+replicase expression system in a plasmid containing Plastocyanine-P19-Plastocyanine expression cassette as follows using the PCR-based ligation method presented by Darveau et al. (Methods in Neuroscience 26: 77-85 (1995)). In a first round of PCR, a fragment containing Rab G ectodomain without the native signal peptide, transmembrane and cytoplasmic domains was amplified using primers IF-RabG-S2+4.c (Figure 8B, SEQ ID NO:32) and RabG+H5dTm.r (Figure 8C, SEQ ID NO: 33), using synthesized Rab G gene (corresponding to nt 3317-4891 from Genebank accession number EF206707) (Figure 8D, SEQ ID NO:34) as template. A second fragment containing the transmembrane and cytoplasmic domains of H5 A/Indonesia/5/2005 was amplified using primers IF-H5dTm.c (Figure 8E, SEQ ID NO: 35) and IF-H5dTm.r (Figure 3C, SEQ ID NO: 15), using construct number 972 (see Figure 94, SEQ ID NO: 134 of WO 2010/003225, , for the sequence of construct number 972) as template. The PCR products from both amplifications were then mixed and used as template for a second round of amplification using IF-RabG-S2+4.c (Figure 8B, SEQ ID NO:32)and IF-H5dTm.r (Figure 3C, SEQ ID NO: 15) as primers. The resulting fragment was cloned in-frame with alfalfa PDI signal peptide in 2X35S-CPMV HT-NOS expression cassette into BeYDV+replicase amplification system using In-Fusion cloning system by Clontech (Mountain View, CA). Construct 144 was digested with SbfI and StuI restriction enzyme and used for the In-Fusion assembly reaction. Construct number 144 is an acceptor plasmid intended for "In Fusion" cloning of genes of interest in a CPMV HT-based expression cassette into the BeYDV amplification system. It also incorporates a gene construct for the co-expression of the TBSV P19 suppressor of silencing under the alfalfa Plastocyanin gene promoter and terminator. The vector is a pCAMBIA-based plasmid and the sequence from left to right t-DNA borders is presented in Figure 8J (SEQ ID NO:39). A schematic representation of the vector 144 is presented in Figure 8K.The resulting construct was given number 1094 (Figure 8L, SEQ ID NO:40). The amino acid sequence of PDISP - Rab G - A/Indonesia/5/2005 H5 TM+CT is presented in Figure 8I (SEQ ID NO:38). The 1094 plasmid representation is presented in Figure 8M. This construct does not encode an M1 protein.

### Example 5: Expression of chimeric Rabies proteins in plants

G protein was expressed in fusion with PDI Sp (construct 1071). Construct 1074 is a fusion of rabies G protein with PDI Sp and H5A/ Indo TM+CT domain. Construct 1094 is a fusion of rabies G protein with BeYDV + rep, PDI Sp and H5A/ Indo TM+CT domain. Construct 1091 is a fusion of rabies G protein with PDI Sp and BeYDV + rep.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct (constructs 1071, 1071, 1074, 1091, and 1094) were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest. Unless otherwise specified, all infiltrations were performed as co-infiltration with strain AGL1/172 in a 1:1 ratio.

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with 0.5ug/ul of a Santa Cruz SE-57995 primary antibody in 2% skim milk in TBS-Tween 20 0.1%. Chemiluminescence detection was carried on after incubation with peroxidase-conjugated goat anti mouse, JIR, 115-035-146 secondary antibody diluted 1:10,000 in 2% skim milk in TBS-Tween 20 0.1%. Immunoreactive complexes were detected by chemiluminescence using luminol as the substrate (Roche Diagnostics Corporation).

As shown in Figure 8N, the rabies G protein was expressed in fusion with PDI Sp (construct 1071), BeYDV + rep (constructs 1094 or 1091), H5A/ Indo TM+CT domain (construct 1074) or a combination thereof.

### Example 6: Chimeric Rabies G protein assemble into virus-like particles

The capacity of the chimeric rabies G protein to assemble into VLP in plant in absence of core or matrix protein was also examined. Protein extracts from plants transformed using construct 1074 or construct 1094, were clarified and subjected to gel filtration chromatography followed by analysis of elution fractions for rabies G content using Santa Cruz SE-57995 primary antibodies. The Western blot presented in Figure 8O shows that extracts from infiltrated plants, chimeric rabies G content in elution fractions peaks in fractions 8 to 14 for protein produced using construct 1074, and fractions a majority of the protein eluting in fractions 8 to 12 for protein extracts prepared using construct 1094, indicating their assembly in very high molecular weight structures of more than 2 million daltons. These results show that rabies G assembles into high molecular weight structures of size beyond that of the expected molecular weight of the homotrimer

Figure 9A shows Immunoblot analysis of the purified rabies G protein expressed from construct 1074. Figure 9B shows a transmission electron microscopy picture of the purified rabies G protein VLP derived from expression of construct 1074 showing VLP morphology.

. Therefore, rabies G accumulated at high levels in agroinfiltrated plants and assembled into virus-like particles in absence of core or matrix protein. These results also demonstrate that the fusion of TM/CT domains of influenza HA to non-influenza antigen, such as rabies G protein, is sufficient to assemble and release VLPs presenting the non-influenza antigen.

### Example 7: Assembly of expression cassettes with SARS

### B-2X35S-CPMV HT-Severe Acute Respiratory Syndrome Virus glycoprotein S (SARS gS)+H5 A/Indonesia/5/2005 transmembrane domain and cytoplasmic tail (TM+CT)-NOS (Construct number 916; Figures 12A, 12F)

A sequence encoding SARS glycoprotein S (SARS gS) ectodomain fused to the transmembrane and cytosolic domains of H5 A/Indonesia/5/2005 was cloned into 2X35S-CPMV-HT expression system as follows using the PCR-based ligation method presented by Darveau et al. (Methods in Neuroscience 26: 77-85 (1995)). In a first round of PCR, a fragment containing SARS gS ectodomainwithout the native transmembrane and cytoplasmic domains was amplified using primers IF-wtSp-SARSgS.c (Figure 12B, SEQ ID NO:26) and IF-H5dTm+SARSgS.r (Figure 12C, SEQ ID NO:27), using synthesized SARS gS gene (corresponding to nt21492-25259 from Genebank accession number AY278741.1; Figure 12D, SEQ ID NO:28) as template. A second fragment containing the transmembrane and cytoplasmic domains of H5 A/Indonesia/5/2005 was amplified using primers SARSgS+H5dTm.c (Figure 12E, SEQ ID NO:29) and IF-H5dTm.r (Figure 3C, SEQ ID NO: 15), using construct number 972 (see Figure 94, SEQ ID NO: 134 of WO 2010/003225, for the sequence of construct number 972) as template. The PCR products from both amplifications were then mixed and used as template for a second round of amplification using IF-wtSp-SARSgS.c (Figure 12B, SEQ ID NO:26) and IF-H5dTm.r (Figure 3C, SEQ ID NO: 15) as primers. The product of the second PCR was then digested with ApaI and StuI and cloned into construct number 995 (Figure 1F, SEQ ID NO: 6) digested with with ApaI and StuI restriction enzymes. The resulting construct was given number 916 (Figure 12F, SEQ ID NO:30). The amino acid sequence of SARS gS - A/Indonesia/5/2005 H5 TM+CT is presented in Figure 12G (SEQ ID NO:31). The 916 plasmid representation is presented in Figure 12A. This construct does not encode an M1 protein.

### Example 8: Expression experiments with chimeric SARS with and without production enhancing factors in plants

SARS proteins were expressed using construct 916 comprising SARS gS gene with wild type signal peptide and H5A/ Indo transmembrane and cytosolic tail domain.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct (construct 916) were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest. Unless otherwise specified, all infiltrations were performed as co-infiltration with strain AGL1/172 in a 1:1 ratio.

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with 2 ug/ul of a Imgenex. IMG-690 primary antibody in 2% skim milk in TBS-Tween 20 0.1%. Chemiluminescence detection was carried on after incubation with peroxidase-conjugated mouse anti-rabbit IgG, JIR, 115-035-144 secondary antibody diluted 1:10,000 in 2% skim milk in TBS-Tween 20 0.1%. Immunoreactive complexes were detected by chemiluminescence using luminol as the substrate (Roche Diagnostics Corporation).

Figure 12H shows Immunoblot analysis of expression of severe acute respiratory syndrome (SARS) virus protein S in tobacco. Expression of construct 916 (SARS gS gene with wild type signal peptide and H5A/ Indo transmembrane and cytosolic tail domain) is observed.

### Example 9: Assembly of expression cassettes with VZV protein

### A-2X35S-CPMV HT-VaricellaZoster Virus glycoprotein E (VZVgE)+H5 A/Indonesia/5/2005 transmembrane domain and cytoplasmic tail (TM+CT)-NOS (Construct number 946)

A sequence encoding VZV glycoprotein E (VZV gE) ectodomain fused to the transmembrane and cytosolic domains of H5 A/Indonesia/5/2005 was cloned into 2X35S-CPMV-HT expression system as follows using the PCR-based ligation method presented by Darveau et al. (Methods in Neuroscience 26: 77-85 (1995)). In a first round of PCR, a fragment containing VZV gE ectodomain without the native transmembrane and cytoplasmic domains was amplified using primers IF-wtSp-VZVgE.c (Figure 10A, SEQ ID NO:20) and IF-H5dTm+VZVgE.r (Figure 10B, SEQ ID NO:21), using synthesized VZV gE gene (corresponding to nt3477-5348 from Genebank accession numberAY013752.1; Figure 10C, SEQ ID NO:22) as template. A second fragment containing the transmembrane and cytoplasmic domains of H5 A/Indonesia/5/2005 was amplified using primers VZVgE+H5dTm.c (Figure 10D, SEQ ID NO:23) and IF-H5dTm.r (Figure 3C, SEQ ID NO: 15), using construct number 972 (see Figure 94, SEQ ID NO: 134 of WO 2010/003225, for the sequence of construct number 972) as template. The PCR products from both amplifications were then mixed and used as template for a second round of amplification using IF-wtSp-VZVgE.c(Figure 10A, SEQ ID NO:20) and IF-H5dTm.r (Figure 3C, SEQ ID NO: 15) as primers. The product of the second PCR was then digested with ApaI and StuI and cloned into construct number 995 (Figure 1F, SEQ ID NO: 6) digested with withApaI and StuI restriction enzymes. The resulting construct was given number 946 (Figure A5, SEQ ID NO: A5).The amino acid sequence of VZV gE- A/Indonesia/5/2005 H5 TM+CT is presented in Figure 10F (SEQ ID NO:25). The 946 plasmid representation is presented in Figure 10G. This construct does not encode an M1 protein.

### Example 10: Expression of chimeric VZV proteins in plants

Expression of Varicella Zoster Virus (VZV) E protein was demonstrated using construct 946, comprising VZV gE gene with wild type signal peptide and H5A/ Indo TM+CT domain.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct (constructs 946) were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6). and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest. Unless otherwise specified, all infiltrations were performed as co-infiltration with strain AGL1/172 in a 1:1 ratio.

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with 1 ug/ul of mouse mAb anti-VZVgE protein (abcam, ab52549) as primary antibody in 2% skim milk in TBS-Tween 20 0.1%. Chemiluminescence detection was carried on after incubation with peroxidase-conjugated goat anti-mouse, JIR, 115-035-146 as secondary antibody diluted 1:10,000 in 2% skim milk in TBS-Tween 20 0.1%. Immunoreactive complexes were detected by chemiluminescence using luminol as the substrate (Roche Diagnostics Corporation).

Figure 11A shows immunoblot analysis of expression of Varicella Zoster Virus (VZV) E protein in Lanes 7-9 from construct 946 (20, 10 and 2 µg of extract respectively). Lanes 1 to 5, positive controls - recombinant VZV gE, 500, 100, 50, 10 and 5 ng, respectively; Lane 6 negative control.

The capacity of the chimeric VZV E protein to assemble into VLP in plant in absence of core or matrix protein was also examined. Protein extracts from plants transformed using construct 946 were clarified and subjected to gel filtration chromatography followed by analysis of elution fractions for VZV E protein using mouse mAb anti-VZVgE protein (abcam, ab52549) primary antibody. The Western blot presented in Figure 11B shows that in extracts from infiltrated plants, chimeric VZV E protein elution fractions peak in fractions 10 to 13, indicating VZV E protein assembly in very high molecular weight structures of more than 2 million daltons. These results show that VZV E protein assembles into high molecular weight structures of size beyond that of the expected molecular weight of the homotrimer.

Therefore, VZV E protein accumulated at high levels in agroinfiltrated plants and assembled into virus-like particles in absence of core or matrix protein. These results also demonstrate that the fusion of TM/CT domains of influenza HA to non-influenza antigen, such as VZV E protein, is sufficient to assemble and release VLPs presenting the non-influenza antigen.

### Example 11. Assembly of expression cassette with chimeric Ebola virus glycoprotein (GP)

### A-2X35S-CPMV HT-PDISP-Zaire Ebolavirus GP (EboGP)+H5 A/Indonesia/5/2005 transmembrane domain and cytoplasmic tail (TM+CT)-NOS (Construct number 1366)

A sequence encoding the ectodomain of Ebola virus glycoprotein (GP) from strain Zaire 1995 fused to the transmembrane and cytosolic domains of H5 A/Indonesia/5/2005 was cloned into 2X35S-CPMV HT-PDISP-NOS expression system in a plasmid containing Plastocyanin-P19-Plastocyanin expression cassette as follows using the PCR-based ligation method presented by Darveau et al. (Methods in Neuroscience 26: 77-85 (1995)). The Ebola GP gene was optimized for codon usage and for GC content from the wild-type gene sequence (corresponding to nt 6039-8069 from GenBank accession number AY354458). Cryptic splice sites, Shine-Delgarno sequences, RNA destabilizing sequences and prokaryotic ribosome entry sites were then remove from optimized sequence to avoid unwanted structure or sequence. In a first round of PCR, a fragment of the optimized GP gene containing the sequence encoding the ectodomain (without the signal peptide and the transmembrane and cytoplasmic domains) was amplified using primers IF-Opt_EboGP.s2+4c (Figure 13A, SEQ ID NO: 43) and H5iTMCT+Opt_EboGP.r (Figure 13B, SEQ ID NO: 44), with the synthesized GP gene (Figure 13C, SEQ ID NO:45) as template. A second fragment containing the transmembrane and cytoplasmic domains of H5 from Influenza A/Indonesia/5/2005 was amplified using primers Opt_EboGP+H5iTMCT.c (Figure 13D, SEQ ID NO: 46) and IF-H5dTm.r (Figure 3C, SEQ ID NO: 15), using construct number 972 (see Figure 94, SEQ ID NO: 134 of WO 2010/003225, for the sequence of construct number 972) as template. The PCR products from both amplifications were then mixed and used as template for a second round of amplification using IF-Opt_EboGP.s2+4c (Figure 13A, SEQ ID NO: 43) and IF-H5dTm.r (Figure 3C, SEQ ID NO: 15) as primers. The resulting PCR product was cloned in-frame with alfalfa PDI signal peptide in 2X35S-CPMV HT-NOS expression system using In-Fusion cloning system by Clontech (Mountain View, CA). Construct 1192 (Figure 13E) was digested with SacII and StuI restriction enzyme and used for the In-Fusion assembly reaction. Construct number 1192 is an acceptor plasmid intended for "In Fusion" cloning of genes of interest in frame with an alfalfa PDI signal peptide in a CPMV HT-based expression cassette. It also incorporates a gene construct for the co-expression of the TBSV P19 suppressor of silencing under the alfalfa Plastocyanin gene promoter and terminator. The vector is a pCAMBIA-based plasmid and the sequence from left to right t-DNA borders is presented in Figure 13F (SEQ ID NO: 47). The resulting construct was given number 1366 (Figure 13G, SEQ ID NO: 48). The amino acid sequence of PDISP-GP from Zaire 95 Ebolavirus-H5 TM+CT from A/Indonesia/5/2005 is presented in Figure 13H (SEQ ID NO: 49). The 1366 plasmid representation is presented in Figure 13I.

### Example 12. Expression of chimeric Ebola virus GP in plants

Expression of Ebola virus (EV) glycoprotein (GP) was demonstrated using construct 1366, comprising EV GP gene with wild type signal peptide and H5A/ Indo TM+CT domain.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct (constructs 946) were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6) and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest.

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 0.15 M NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analyses.

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with 150 ng/ml of affinity purified rabbit anti-Ebola GP Zaire (IBT Bioservices, 0301-012) as primary antibody in 2% skim milk in TBS-Tween 20 0.1%. Chemiluminescence detection was carried on after incubation with peroxidase-conjugated goat anti-rabbit secondary antibodies (JIR 11-035-144), diluted 1:7500 in 2% skim milk in TBS-Tween 20 0.1%. Immunoreactive complexes were detected by chemiluminescence using luminol as the substrate (Roche Diagnostics Corporation). Figure 13J shows immunoblot analysis of expression of chimeric Ebola virus GP in protein extracts from plants transformed with construct number 1366. The result obtained shows that chimeric Ebola GP is transiently expressed.

## Claims

1. A method of producing a virus like particle (VLP) in a plant comprising,
a) introducing a nucleic acid comprising a regulatory region active in the plant and operatively linked to a chimeric nucleotide sequence encoding, in series, an ectodomain from a virus trimeric surface protein fused to an influenza transmembrane domain and cytoplasmic tail domain (TM/CT), into the plant, or portion of the plant, wherein the TM/CT is from an influenza hemagglutinin and the ectodomain is derived from a virus of a family of Retroviridae, Rhabdoviridae, Coronaviridae or Filoviridae, and
b) incubating the plant or portion of the plant under conditions that permit the expression of the nucleic acid, thereby producing the VLP, the VLP comprising viral proteins, wherein the viral proteins consist of chimeric viral surface proteins.

2. The method of claim 1, wherein the ectodomain from the virus trimeric surface protein is derived from the genus Lentivirus, Lyssavirus, Coronavirus or Ebolavirus.

3. The method of claim 1, wherein the ectodomain from the virus trimeric surface protein is derived from Human immunodeficiency virus (HIV), Rabies virus, Severe acute respiratory syndrome (SARS) virus, or Ebola virus.

4. The method of claim 1, wherein the ectodomain from the virus trimeric surface protein is derived from F protein, S protein, env protein, G protein, a E envelope glycoprotein, B envelope glycoprotein, C envelope glycoprotein, I envelope glycoprotein, H envelope glycoprotein, GP glycoprotein, or hemagglutinin.

5. The method of claim 1, wherein in the step of introducing (step a), the nucleic acid is transiently expressed in the plant.

6. The method of claim 1, wherein, in the step of introducing (step a), the nucleic acid is stably expressed in the plant.

7. The method of claim 1, further comprising a step of:
c) harvesting the plant and purifying the VLP.

8. The method of claim 1, wherein, in the step of introducing (step a), one or more than one additional nucleic acid, selected from the group of a nucleotide sequence encoding one or more than one chaperone protein, proton channel protein, protease inhibitor, or a combination thereof, is introduced to the plant.

9. The method of claim 1, wherein the VLP does not contain a viral matrix or a core protein.

10. The method of claim 1, wherein in the step of introducing (step a), the influenza transmembrane domain and cytoplasmic tail domain is an H5 transmembrane domain and cytoplasmic tail domain, or an H1 transmembrane domain and cytoplasmic tail domain, or an H3 transmembrane domain and cytoplasmic tail domain.

11. A VLP produced by the method of claim 1 or claim 10.

12. The VLP of claim 11, comprising a chimeric virus protein bearing plant-specific N-glycans, or modified N-glycans.

13. The VLP of claim 11, comprising one or more than one lipid derived from the plant.

14. A composition comprising an effective dose of the VLP of claim 11 for inducing an immune response, and a pharmaceutically acceptable carrier.

15. The method of claim 1 wherein the influenza transmembrane domain and cytoplasmic tail domain is obtained from H5 (A/Indonesia/05/2005) and comprises the nucleotide sequence defined in SEQ ID NO:41, or the influenza transmembrane domain and cytoplasmic tail domain is obtained from H3 (A/Brisbane/10/2007) and comprises the sequence defined in SEQ ID NO:42.

## Patentansprüche

1. Verfahren zur Produktion eines virusähnlichen Partikels (VLP) in einer Pflanze, umfassend,
a) Einführen einer Nukleinsäure, umfassend eine regulatorische Region, die in der Pflanze aktiv ist, und in Wirkbeziehung mit einer chimären Nukleotidsequenz verknüpft ist, die der Reihe nach für eine Ektodomäne eines trimeren Virusoberflächenproteins fusioniert an eine Influenza-Transmembrandomäne und eine zytoplasmatische Schwanzdomäne (TM/CT) codiert, in die Pflanze oder einen Teil der Pflanze, wobei die TM/CT aus einem Influenza-Hämagglutinin stammt und die Ektodomäne von einem Virus einer Familie von Retroviridae, Rhabdoviridae, Coronaviridae oder Filoviridae abgeleitet ist und
b) Inkubieren der Pflanze oder eines Teils der Pflanze unter Bedingungen, die die Expression der Nukleinsäure ermöglichen, wodurch das VLP produziert wird, wobei das VLP Virusproteine umfasst, wobei die Virusproteine aus chimären viralen Oberflächenproteinen bestehen.

2. Verfahren nach Anspruch 1, wobei die Ektodomäne aus dem trimeren Virusoberflächenprotein aus der Gattung Lentivirus, Lyssavirus, Coronavirus oder Ebolavirus abgeleitet wird.

3. Verfahren nach Anspruch 1, wobei die Ektodomäne aus dem trimeren Virusoberflächenprotein aus dem humanen Immundefizienz-Virus (HIV), Tollwutvirus, Schweres-akutesrespiratorisches-Syndrom (SARS)-Virus oder Ebola-Virus abgeleitet ist.

4. Verfahren nach Anspruch 1, wobei die Ektodomäne aus dem trimeren Virusoberflächenprotein von F-Protein, S-Protein env-Protein, G-Protein, einem E-Hüllglykoprotein, B-Hüllglykoprotein, C-Hüllglykoprotein, I-Hüllglykoprotein, H-Hüllglykoprotein, GP-Glycoprotein oder Hämagglutinin abgeleitet ist.

5. Verfahren nach Anspruch 1, wobei bei dem Schritt des Einführens (Schritt a) die Nukleinsäure in der Pflanze transient exprimiert wird.

6. Verfahren nach Anspruch 1, wobei bei dem Schritt (Schritt a) des Einführens die Nukleinsäure in der Pflanze stabil exprimiert wird.

7. Verfahren nach Anspruch 1, weiter umfassend einen Schritt des:
c) Erntens der Pflanze und das Aufreinigen des VLP.

8. Verfahren nach Anspruch 1, wobei in dem Schritt des Einführens (Schritt a) eine oder mehr als eine weitere Nukleinsäuren, ausgewählt aus der Gruppe aus einer Nukleotidsequenz, die für ein bzw. einen oder mehr als ein Chaperon-Protein, Protonkanalprotein, Protease-Inhibitor oder eine Kombination davon codiert, in die Pflanze eingeführt wird.

9. Verfahren nach Anspruch 1, wobei das VLP keine virale Matrix oder ein Kernprotein enthält.

10. Verfahren nach Anspruch 1, wobei bei dem Schritt des Einführens (Schritt a) die Influenza-Transmembrandomäne und die Zytoplasmaschwanzdomäne eine H5-Transmembrandomäne und eine Zytoplasmaschwanzdomäne oder eine H1-Transmembrandomäne und eine Zytoplasmaschwanzdomäne oder eine H3-Transmembrandomäne und eine Zytoplasmaschwanzdomäne sind.

11. VLP, erzeugt nach dem Verfahren von Anspruch 1 oder Anspruch 10.

12. VLP nach Anspruch 11, umfassend ein chimäres Virusprotein, das pflanzenspezifische N-Glykane oder modifizierte N-Glykane trägt.

13. VLP nach Anspruch 11, umfassend ein oder mehrere aus der Pflanze abgeleitete Lipide.

14. Zusammensetzung, umfassend eine wirksame Dosis des VLP nach Anspruch 11 zum Induzieren einer Immunantwort und einen pharmazeutisch annehmbaren Träger.

15. Verfahren nach Anspruch 1, wobei die Influenza-Transmembrandomäne und Zytoplasmaschwanzdomäne aus H5 (A/Indonesien/05/2005) erhalten werden und die in SEQ ID NO: 41 definierte Nukleotidsequenz umfassen oder die Influenza-Transmembrandomäne und Zytoplasmaschwanzdomäne aus H3 (A/Brisbane/10/2007) erhalten werden und die in SEQ ID NO: 42 definierte Sequenz umfassen.

## Revendications

1. Procédé de production d'une pseudo-particule virale (PPV) dans une plante comprenant,
a) l'introduction d'un acide nucléique comprenant une région régulatrice active dans la plante et lié à une séquence nucléotidique chimérique codant, en série, pour un ectodomaine provenant d'une protéine de surface trimère virale fusionnée à un domaine transmembranaire et domaine de queue cytoplasmique (TM/CT) du virus de la grippe, dans la plante, ou une partie de la plante, dans lequel le TM/CT est issu d'une hémagglutinine du virus de la grippe et l'ectodomaine est dérivé d'un virus de la famille des Rétroviridés, des Rhabdoviridés, des Coronaviridés ou des Filoviridés, et
b) l'incubation de la plante ou d'une partie de la plante dans des conditions qui permettent l'expression de l'acide nucléique, produisant ainsi la PPV, la PPV comprenant des protéines virales, les protéines virales étant constituées de protéines de surface virales chimériques.

2. Procédé selon la revendication 1, dans lequel l'ectodomaine issu de la protéine de surface trimère virale est dérivé du genre Lentivirus, Lyssavirus, Coronavirus ou Ebolavirus.

3. Procédé selon la revendication 1, dans lequel l'ectodomaine issu de la protéine de surface trimère virale est dérivé du virus de l'immunodéficience humaine (VIH), du virus de la rage, du virus du syndrome respiratoire aigu sévère (SRAS), ou du virus Ebola.

4. Procédé selon la revendication 1, dans lequel l'ectodomaine issu de la protéine de surface trimère virale est dérivé de la protéine F, de la protéine S, de la protéine env, de la protéine G, d'une glycoprotéine de l'enveloppe E, d'une glycoprotéine de l'enveloppe B, d'une glycoprotéine de l'enveloppe C, d'une glycoprotéine de l'enveloppe I, d'une glycoprotéine de l'enveloppe H, d'une glycoprotéine GP, ou de l'hémagglutinine.

5. Procédé selon la revendication 1, dans lequel dans l'étape d'introduction (étape a), l'acide nucléique est exprimé de façon transitoire dans la plante.

6. Procédé selon la revendication 1, dans lequel, dans l'étape d'introduction (étape a), l'acide nucléique est exprimé de façon stable dans la plante.

7. Procédé selon la revendication 1, comprenant en outre une étape de :
c) récolte de la plante et purification de la PPV.

8. Procédé selon la revendication 1, dans lequel, dans l'étape d'introduction (étape a), un ou plus d'un acide nucléique supplémentaire, choisi dans le groupe d'une séquence nucléotidique codant pour une ou plus d'une protéine chaperon, protéine de canal à protons, inhibiteur de protéase, ou l'une de leurs combinaisons, est introduit dans la plante.

9. Procédé selon la revendication 1, dans lequel la PPV ne contient pas de matrice virale ni de protéine nucléocapsidique.

10. Procédé selon la revendication 1, dans lequel dans l'étape d'introduction (étape a), le domaine transmembranaire et domaine de queue cytoplasmique du virus de la grippe consistent en un domaine transmembranaire et domaine de queue cytoplasmique H5, ou un domaine transmembranaire et domaine de queue cytoplasmique H1, ou un domaine transmembranaire et domaine de queue cytoplasmique H3.

11. PPV produite par le procédé selon la revendication 1 ou la revendication 10.

12. PPV selon la revendication 11, comprenant une protéine virale chimérique portant des N-glycanes spécifiques de la plante, ou des N-glycanes modifiés.

13. PPV selon la revendication 11, comprenant un ou plus d'un lipide dérivé de la plante.

14. Composition comprenant une dose efficace de la PPV selon la revendication 11 pour l'induction d'une réponse immunitaire, et un support pharmaceutiquement acceptable.

15. Procédé selon la revendication 1, dans lequel le domaine transmembranaire et domaine de queue cytoplasmique du virus de la grippe sont obtenus à partir de H5 (A/Indonesia/05/2005) et comprennent la séquence nucléotidique définie dans SEQIDNO:41, ou le domaine transmembranaire et domaine de queue cytoplasmique du virus de la grippe sont obtenus à partir de H3 (A/Brisbane/10/2007) et comprennent la séquence nucléotidique définie dans SEQ ID NO : 42.
